(19) Europäisches Patentamt
European Patent Office
Office européen des brevets

(11) Publication number: **0 602 242 A1**

# (12) EUROPEAN PATENT APPLICATION published in accordance with Art. 158(3) EPC

(21) Application number: **92918032.1**

(22) Date of filing: **21.08.92**

(86) International application number: **PCT/JP92/01060**

(87) International publication number: **WO 93/04063 (04.03.93 93/06)**

(51) Int. Cl.5: **C07D 413/06**, C07D 413/12, C07D 413/14, C07D 498/04, A61K 31/41, A61K 31/445, A61K 31/535, A61K 31/55

(30) Priority: **22.08.91 JP 237397/91**
**25.03.92 JP 100367/92**
**26.03.92 JP 101707/92**
**26.03.92 JP 101763/92**
**03.04.92 JP 112154/92**

(43) Date of publication of application: **22.06.94 Bulletin 94/25**

(84) Designated Contracting States: **AT BE CH DE DK ES FR GB GR IE IT LI LU MC NL SE**

(71) Applicant: **YOSHITOMI PHARMACEUTICAL INDUSTRIES, LTD.**
**6-9, Hiranomachi 2-chome**
**Chuo-ku**
**Osaka-shi Osaka 541(JP)**

(72) Inventor: **SUEOKA, Hiroyuki Yoshitomi Pharmac. Ind. Ltd.**
**Central Res. Labs.**
**955 Oaza-Koiwai**
**Yoshitomimachi**
**Chikujo-gun Fukuoka 871(JP)**
Inventor: **MURAKAMI, Shu Yoshitomi Pharmaceutical Ind. Ltd.**
**Central Res. Labs.**
**955 Oaza-Koiwai**
**Yoshitomimachi**
**Chikujio-gun Fukuoka 871(JP)**
Inventor: **TAKEHARA, Shuzo Yoshitomi Pharmaceutical Ind. Ltd.**
**Central Res. Labs.**
**955 Oaza-koiwai**
**Yoshitomimachi**
**Chikujo-gun Fukuoka 871(JP)**

(74) Representative: **von Kreisler, Alek, Dipl.-Chem. et al**
**Patentanwälte**
**von Kreisler-Selting-Werner**
**Bahnhofsvorplatz 1 (Deichmannhaus)**
**D-50667 Köln (DE)**

## (54) BENZISOXAZOLE COMPOUND AND USE THEREOF.

(57) A benzisoxazole compound represented by general formula (I) or a pharmaceutically acceptable salt thereof wherein, when the bond (a) between the 2- and 3-positions represents a single bond, $R_a$ represents the group (II) (wherein R represents hydrogen, alkyl, alkenyl, cycloalkylalkyl, cycloalkylalkenyl, phenylalkenyl, naphthylalkyl or naphthylalkenyl; A represents linear or branched alkylene; and n represents 1, 2 or 3) and $R_b$ represents oxygen, or alternatively when the bond (a) between the 2- and 3-positions represents a double bond, $R_a$ is absent; $R_b$ represents either the group (III), wherein each symbol is as defined above, or the group (IV), wherein E represents oxygen or sulfur, and R, A and n are each as defined above; and $R^1$, $R^2$, $R^3$ and $R^4$ are each as defined in the specification. This compound has a centroselective acetylcholine esterase inhibiting activity and/or a potent affinity for sigma receptor.

EP 0 602 242 A1

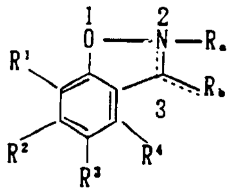 (I)

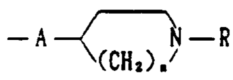 (II)

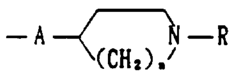 (III)

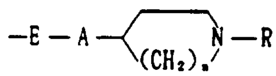 (IV)

## Technical Field

The present invention relates to a novel benzisoxazole compound useful as a pharmaceutical and a synthetic intermediate for a pharmaceutical, a pharmaceutically acceptable salt thereof and pharmaceutical use thereof.

## Background Art

With the advent of an aging society, patients of senile dementia such as Alzheimer's disease and cerebrovascular dementia have increased in number and therapeutic agents therefor are desired. So as to meet the need, brain function-improving agents and antidementia have been studied from various aspects. One attempt is to treat the diseases by enhancing the function of acetylcholine system in the central nervous system Of the agents to this effect, acetylcholinesterase inhibitors such as physostigmine and tetrahydroaminoacridine (THA) are known to have an improving action on brain function [Drug. Dev. Res., Vol. 5, p. 77 (1985)]. Acetylcholine, however, manifests various physiological actions not only in the central nervous system but also in the periphery. For example, it acts on secretory glands to promote various secretions such as slobbering, delacrimation, perspiration and so on and it acts on circulartory system to cause dilation of peripheral blood vessels, leading to lowered blood pressure. It follows therefrom that the center-selectivity should be very important when an acetylcholinesterase inhibitor is used as a brain function-improving agent or antidementia.

Traditionally, the drug treatment for psychosis has been based on the so-called hyper-dopamine (DA) theory and $D_2$ receptor-blocking agents represented by haloperidol and chlorpromazine have been used in clinical fields. However, there exist some patients who fail to show improvement with these drugs and these drugs are all but ineffective for negative symptoms. Accordingly, the hyper-DA theory has been gradually considered to be not a theory applicable to all schizophrenia but one hypothesis to clarify positive symptoms such as hallucination and delusion. In addition, these drugs are associated with an incidence of side effects such as extrapyramidal disorders caused by blocking of the DA receptor in the substantia nigra - striatum system. For this reason, elucidation of a new drug receptor concerned with an antipsychotic action has been demanded. As a result of the studies of conventionally-employed drugs such as antipsychotic and antidepressant which act on the central nervous system, the presence of a drug receptor commonly acted on by these drugs has been gradually recognized [Pharmacological Reviews, Vol. 42, 4, p. 355 (1990)], namely, a sigma receptor. A pharmaceutical having affinity for said receptor is expected to be efficacious for central diseases such as psychosis, depression, anxiety and so on. It is also considered that said receptor distributes widely not only in the central nervous system but also in the periphery and plays an important role in psychic control, internal secretion, metabolism etc., thus producing expectation for its effect on various diseases in the periphery. Yet, known drugs having affinity for sigma receptor fail to exhibit satisfactory properties in terms of strength, selectivity and duration of the affinity for said receptor.

Incidentally, Japanese Patent Unexamined Publication No. 221186/1986 discloses benzisoxazole derivatives having antipsychotic action, J. Med. Chem., Vol. 25, p. 36 (1982) discloses benzisoxaxole derivatives having diuretic action and Japanese Patent Unexamined Publication No. 79151/1989 discloses cyclic amine derivatives having acetylcholinesterase inhibitory action.

Also, EP-A-449,186, EP-A-449,187, WO91/06297 and Japanese Patent Unexamined Publication No. 59775/1992 disclose compounds having a piperidine ring and affinity for the sigma receptor.

## Disclosure of the Invention

In view of the aforementioned aspects, the present inventors have conducted intensive studies and found that a novel benzisoxazole compound has a center-selective, acetylcholinesterase inhibitory action and/or potent affinity for the sigma receptor and that the compound is superior in selectivity and duration, which resulted in the completion of the invention. The present invention further provides a novel synthetic intermediate thereof.

That is, the present invention relates to

(1) a benzisoxazole compound of the formula

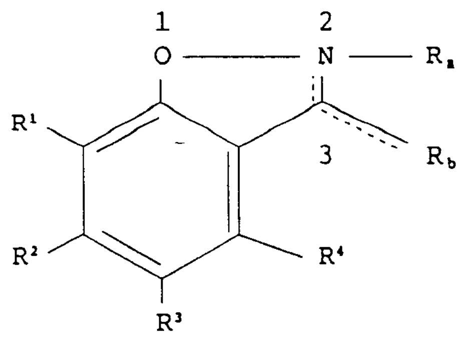

(I)

or a pharmaceutically acceptable salt thereof wherein: when -------- bond between 2- and 3-positions is a single bond,

$R_a$ is a group of the formula

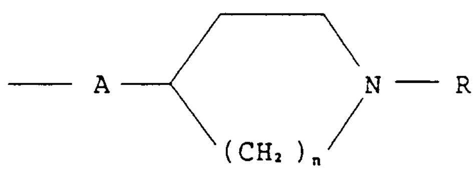

(a)

[wherein:

R is hydrogen, alkyl, alkenyl, cycloalkylalkyl, cycloalkylalkenyl, phenylalkyl, phenylalkenyl, naphthylalkyl or naphthylalkenyl,

A is a straight- or branched chain alkylene, and

n is 1, 2 or 3]; and

$R_b$ is oxygen, and

when -------- bond between 2- and 3-positions is a double bond,

$R_a$ is void; and

$R_b$ is a group of the formula

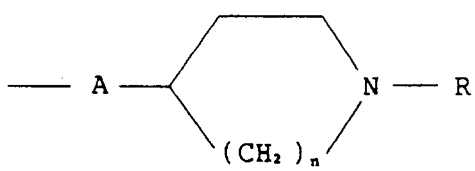

(a)

[wherein each symbol is as defined above], or a group of the formula

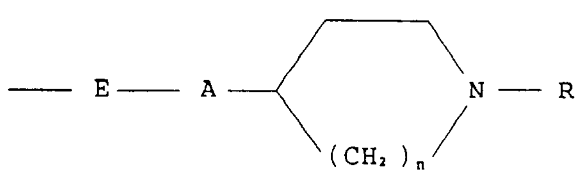

(b)

[wherein:

E is oxygen or sulfur and

other symbols are as defined above]; and

$R^1$, $R^2$, $R^3$ and $R^4$

are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ or

$-COOR^8$ or adjacent groups from $R^1$, $R^2$, $R^3$ and $R^4$ may be bonded to form $-O-(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted,
[wherein:
$R^5$ is alkyl, phenyl or phenylalkyl,
$R^6$ and $R^7$ are the same or different and each is hydrogen, alkyl, phenyl, phenylalkyl or a group bonded with the adjacent nitrogen atom to form a heterocyclic ring,
$R^8$ is hydrogen, alkyl, phenyl or phenylalkyl,
$R^9$ is hydrogen, alkyl, phenylalkyl or acyl,
m is 0, 1 or 2, and
p, q, r and s are the same or different and each is 1, 2 or 3];

wherein:
in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ and $-COOR^8$ [wherein $R^5$, $R^6$, $R^7$, $R^8$ and m are as defined above]:

(2) a benzisoxazole compound of the above (1) or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl:

(3) a benzisoxazole compound of the above (1) or a pharmaceutically acceptable salt thereof, wherein R is hydrogen:

(4) a benzisoxazole compound of the above (1) or a pharmaceutically acceptable salt thereof, having the formula

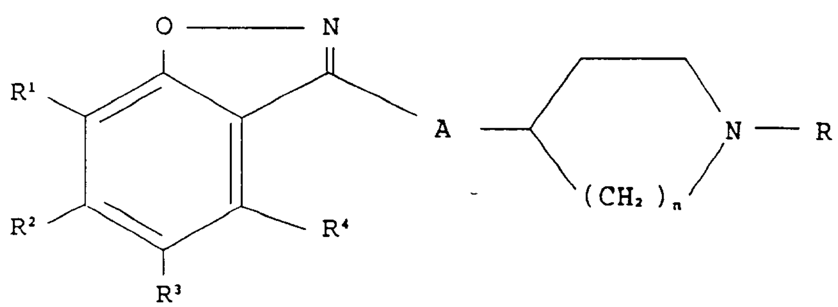

(II)

wherein:

R is hydrogen, alkyl, alkenyl, cycloalkylalkyl, cycloalkylalkenyl, phenylalkyl, phenylalkenyl, naphthylalkyl or naphthylalkenyl;

$R^1$, $R^2$, $R^3$ and $R^4$ are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ or $-COOR^8$, or adjacent groups from $R^1$, $R^2$, $R^3$ and $R^4$ may be bonded to form $-O-(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted
[wherein:
$R^5$ is alkyl, phenyl or phenylalkyl,
$R^6$ and $R^7$ are the same or different and each is hydrogen, alkyl, phenyl, phenylalkyl or a group bonded with the adjacent nitrogen atom to form a heterocyclic ring,
$R^8$ is hydrogen, alkyl, phenyl or phenylalkyl,
$R^9$ is hydrogen, alkyl, phenylalkyl or acyl,
m is 0, 1 or 2, and

p, q, r and s are the same or different and each is 1, 2 or 3];

A is a straight- or branched chain alkylene; and

n is 1, 2 or 3;

wherein:

in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ and $-COOR^8$ [wherein $R^5$, $R^6$, $R^7$, $R^8$ and m are as defined above]:

(5) a benzisoxazole compound of the above (4) or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl:

(6) a benzisoxazole compound of the above (4) or a pharmaceutically acceptable salt thereof, wherein R is hydrogen:

(7) a benzisoxazole compound of the above (1) or a pharmaceutically acceptable salt thereof, having the formula

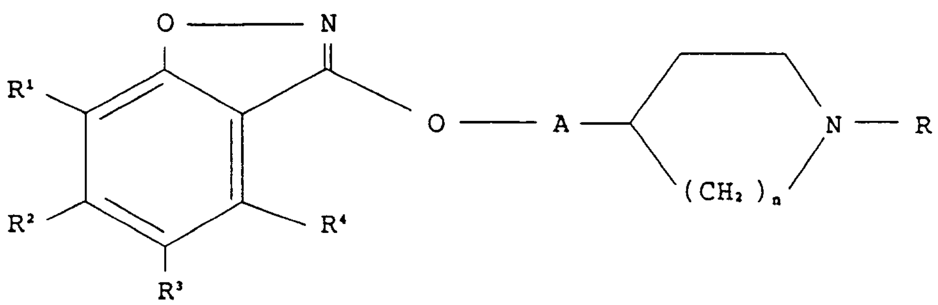

(III)

wherein:

R is hydrogen, alkyl, alkenyl, cycloalkylalkyl, cycloalkylalkenyl, phenylalkyl, phenylalkenyl, naphthylalkyl or naphthylalkenyl;

$R^1$, $R^2$, $R^3$ and $R^4$ are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ or $-COOR^8$, or adjacent groups from $R^1$, $R^2$, $R^3$ and $R^4$ may be bonded to form $-O-(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted,

[wherein:

$R^5$ is alkyl, phenyl or phenylalkyl,

$R^6$ and $R^7$ are the same or different and each is hydrogen, alkyl, phenyl, phenylalkyl or a group bonded with the adjacent nitrogen atom to form a heterocyclic ring,

$R^8$ is hydrogen, alkyl, phenyl or phenylalkyl,

$R^9$ is hydrogen, alkyl, phenylalkyl or acyl,

m is 0, 1 or 2, and

p, q, r and s are the same or different and each is 1, 2 or 3];

A is a straight- or branched alkylene; and

n is 1, 2 or 3;

wherein:

in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ and $-COOR^8$ [wherein $R^5$, $R^6$, $R^7$, $R^8$ and m are as defined above]:

(8) a benzisoxazole compound of the above (7) or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl:

(9) a benzisoxazole compound of the above (7) or a pharmaceutically acceptable salt thereof, wherein R is hydrogen:

(10) a benzisoxazole compound of the above (1) or a pharmaceutically acceptable salt thereof, having the formula

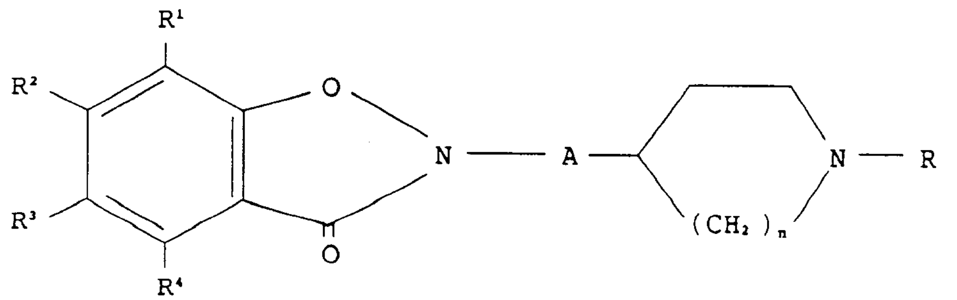

(IV)

wherein:

| | |
|---|---|
| R | is hydrogen, alkyl, alkenyl, cycloalkylalkyl, cycloalkylalkenyl, phenylalkyl, phenylalkenyl, naphthylalkyl or naphthylalkenyl; |
| $R^1$, $R^2$, $R^3$ and $R^4$ | are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ or $-COOR^8$, or adjacent groups from $R^1$, $R^2$, $R^3$ and $R^4$ may be bonded to form $-O-(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted, [wherein: $R^5$ is alkyl, phenyl or phenylalkyl, $R^6$ and $R^7$ are the same or different and each is hydrogen, alkyl, phenyl, phenylalkyl or a group bonded with the adjacent nitrogen atom to form a heterocyclic ring, $R^8$ is hydrogen, alkyl, phenyl or phenylalkyl, $R^9$ is hydrogen, alkyl, phenylalkyl or acyl, m is 0, 1 or 2, and p, q, r and s are the same or different and each is 1, 2 or 3]; |
| A | is a straight- or branched chain alkylene; and |
| n | is 1, 2 or 3; |

wherein:

in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ and $-COOR^8$ [wherein $R^5$, $R^6$, $R^7$, $R^8$ and m are as defined above]:

(11) a benzisoxazole compound of the above (10) or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl:

(12) a benzisoxazole compound of the above (10) or a pharmaceutically acceptable salt thereof, wherein R is hydrogen:

(13) a pharmaceutical composition containing the benzisoxazole compound of the above (2) or its pharmaceutically acceptable salt as an active ingredient:

(14) a pharmaceutical composition containing the benzisoxazole compound of the above (5) or its pharmaceutically acceptable salt as an active ingredient:

(15) a pharmaceutical composition containing the benzisoxazole compound of the above (8) or its pharmaceutically acceptable salt as an active ingredient:

(16) a pharmaceutical composition containing the benzisoxazole compound of the above (11) or its pharmaceutically acceptable salt as an active ingredient:
(17) an acetylcholinesterase inhibitor containing the benzisoxazole compound of the above (5) or its pharmaceutically acceptable salt as an active ingredient:
(18) an acetylcholinesterase inhibitor containing the benzisoxazole compound of the above (8) or its pharmaceutically acceptable salt as an active ingredient:
(19) an acetylcholinesterase inhibitor containing the benzisoxazole compound of the above (11) or its pharmaceutically acceptable salt as an active ingredient:
(20) an agent for the central nervous system, containing the benzisoxazole compound of the above (5) or its pharmaceutically acceptable salt as an active ingredient:
(21) an agent for the center, containing the benzisoxazole compound of the above (8) or its pharmaceutically acceptable salt as an active ingredient: and
(21) an agent for the central nervous system, containing the benzisoxazole compound of the above (11) or its pharmaceutically acceptable salt as an active ingredient.

Of the compounds of the above (5), preferred are
3-(2-(1-benzyl-4-piperidyl)ethyl)-6,7-dimethoxy-1,2-benzisoxazole,
3-(2-(1-(3-(N-methylcarbamoyloxy)benzyl-4-piperidyl)ethyl)-6,7-dimethoxy-1,2-benzisoxazole,
6-acetylamino-3-(2-(1-benzyl-4-piperidyl)ethyl)-1,2-benzisoxazole,
3-(2-(1-benzyl-4-piperidyl)ethyl)-6-methoxy-1,2-benzisoxazole,
3-(2-(1-benzyl-4-piperidyl)ethyl)-5-methoxy-1,2-benzisoxazole,
3-(2-(1-benzyl-4-piperidyl)ethyl)-6-(N,N-dimethylthiocarbamoyloxy)-1,2-benzisoxazole,
3-(2-(1-cyclohexylmethyl-4-piperidyl)ethyl)-6,7-dimethoxy-1,2-benzisoxazole,
3-(2-(1-benzyl-4-piperidyl)ethyl)-6-propionylamino-1,2-benzisoxazole,
3-(2-(1-benzyl-4-piperidyl)ethyl)-6-(N-methylacetamino)-1,2-benzisoxazole,
6-(N,N-dimethylamino)-3-(2-(1-benzyl-4-piperidyl)ethyl-1,2-benzisoxazole,
3-(2-(1-benzyl-4-piperidyl)ethyl)-6-methylamino-1,2-benzisoxazole,
3-(2-(1-(2-chlorobenzyl)-4-piperidyl)ethyl)-6,7-dimethoxy-1,2-benzisoxazole,
3-(2-(1-(4-chlorobenzyl)-4-piperidyl)ethyl)-6,7-dimethoxy-1,2-benzisoxazole,
3-(2-(1-benzyl-4-piperidyl)ethyl)-6-(N,N-dimethylcarbamoyloxy)-1,2-benzisoxazole,
6-acetoxy-3-(2-(1-benzyl-4-piperidyl)ethyl)-1,2-benzisoxazole,
3-(2-(1-benzyl-4-piperidyl)ethyl)-6-(N-methylcarbamoyloxy)-1,2-benzisoxazole,
3-(2-(1-(2-phenylethyl)-4-piperidyl)ethyl)-6,7-dimethoxy-1,2-benzisoxazole,
3-(2-(1-benzyl-4-piperidyl)ethyl)-6-(N-methyl-N-propionylamino)-1,2-benzisoxazole,
6-benzoylamino-3-(2-(1-benzyl-4-piperidyl)ethyl)-1,2-benzisoxazole
and pharmaceutically acceptable salts thereof; of the compounds of the above (8), preferred are
3-(2-(1-cyclohexylmethyl-4-piperidyl)ethyloxy)-5-methyl-1,2-benzisoxazole,
3-(2-(1-(2-phenylethyl)-4-piperidyl)ethyloxy)-1,2-benzisoxazole,
3-(2-(1-cyclohexylmethyl-4-piperidyl)ethyloxy)-6-methyl-1,2-benzisoxazole,
5-methyl-3-(2-(1-(2-phenylethyl)-4-piperidyl)ethyloxy)-1,2-benzisoxazole,
6-chloro-3-(2-(1-(2-phenylethyl)-4-piperidyl)ethyloxy)-1,2-benzisoxazole,
6-methyl-3-(2-(1-(2-phenylethyl)-4-piperidyl)ethyloxy)-1,2-benzisoxazole,
5-methyl-3-(2-(1-benzyl-4-piperidyl)ethyloxy)-1,2-benzisoxazole,
3-(3-(1-benzyl-4-piperidyl)propyloxy)-1,2-benzisoxazole,
3-(2-(1-benzyl-4-piperidyl)ethyloxy)-1,2-benzisoxazole,
3-(2-(1-benzyl-4-piperidyl)ethyloxy)-1,2-benzisoxazole and pharmaceutically acceptable salts thereof; and of the compounds of the above (11), preferred are
2-(2-(1-cyclohexylmethyl-4-piperidyl)ethyl)-5-methyl-1,2-benzisoxazol-3(2H)-one,
5-methyl-2-(2-(1-(2-phenylethyl)-4-piperidyl)ethyl)-1,2-benzisoxazol-3(2H)-one,
2-(2-(1-cyclohexylmethyl-4-piperidyl)ethyl)-6-methyl-1,2-benzisoxazol-3(2H)-one,
6-methyl-2-(2-(1-(2-phenylethyl)-4-piperidyl)ethyl)-1,2-benzisoxazol-3(2H)-one,
6-benzoylamino-2-(2-(1-benzyl-4-piperidyl)ethyl)-1,2-benzisoxazol-3(2H)-one,
6-benzoylamino-2-(3-(1-benzyl-4-piperidyl)propyl)-1,2-benzisoxazol-3(2H)-one and pharmaceutically acceptable salts thereof.

In the definitions of each symbol in the present specification, halogen means chlorine, bromine, fluorine or iodine; alkyl is a straight- or branched chain alkyl having 1 to 10 carbon atoms, such as methyl, ethyl, propyl, isopropyl, butyl, isobutyl, tert-butyl, pentyl, hexyl, 2-ethylhexyl, octyl, 1,1,3,3-tetramethylbutyl and decyl; alkenyl is an alkenyl having 2 to 8 carbon atoms, such as ethenyl, propenyl (e.g. allyl, 1-propenyl), butenyl (e.g. 1-butenyl, 2-butenyl, 3-butenyl), 3,3-dimethylallyl, pentenyl, hexenyl and octenyl; alkoxy is a

straight- or branched chain alkoxy having 1 to 10 carbon atoms, such as methoxy, ethoxy, propoxy, isopropoxy, butoxy, isobutoxy, tert-butoxy, pentyloxy, hexyloxy, 2-ethylhexyloxy, octyloxy and decyloxy; phenylalkyl is that wherein an alkyl moiety is a straight- or branched chain alkyl having 1 to 6 carbon atoms such as methyl, ethyl, propyl, isopropyl, butyl, pentyl and hexyl, which is exemplified by benzyl, 1- or 2-phenylethyl, phenylpropyl, 1-methylphenylethyl, phenylbutyl, phenylpentyl and phenylhexyl; naphthylalkyl is that wherein an alkyl moiety is a straight- or branched chain alkyl having 1 to 6 carbon atoms such as methyl, ethyl, propyl, isopropyl, butyl, pentyl and hexyl, which is exemplified by 1-naphthylmethyl, 2-naphthylmethyl, 2-(1-naphthyl)ethyl, 2-(2-naphthyl)ethyl, 1-(1-naphthyl)ethyl and 1-(2-naphthyl)ethyl; cycloalkylalkyl is that wherein a cycloalkyl moiety is a cycloalkyl having 3 to 7 carbon atoms such as cyclopropyl, cyclobutyl, cyclopentyl, cyclohexyl and cycloheptyl and an alkyl moiety is a straight- or branched chain alkyl having 1 to 6 carbon atoms such as methyl, ethyl, propyl, isopropyl, butyl, pentyl and hexyl, which is exemplified by cyclopropylmethyl, cyclobutylmethyl, cyclopentylmethyl, cyclohexylmethyl, cycloheptylmethyl, cyclopropylethyl, cyclopentylethyl, cyclohexylethyl, cycloheptylethyl, cyclopropylpropyl, cyclopentylpropyl, cyclohexylpropyl, cycloheptylpropyl, cyclopropylbutyl, cyclopentylbutyl, cyclohexylbutyl, cycloheptylbutyl, cyclopropylhexyl, cyclopentylhexyl, cyclohexylhexyl and cycloheptylhexyl; phenylalkenyl is that wherein an alkenyl moiety is an alkenyl having 2 to 8 carbon atoms such as ethenyl, propenyl (e.g. allyl, 1-propenyl), butenyl (e.g. 1-butenyl, 2-butenyl, 3-butenyl) and 3,3-dimethylallyl, pentenyl, hexenyl, octenyl), which is exemplified by styryl, cinnamyl, phenylbutenyl, phenylpentenyl, phenylhexenyl and phenyloctenyl; naphthylalkenyl is that wherein an alkenyl moiety is an alkenyl having 2 to 8 carbon atoms such as ethenyl, propenyl (e.g. allyl, 1-propenyl), butenyl (e.g. 1-butenyl, 2-butenyl, 3-butenyl), 3,3-dimethylallyl, pentenyl, hexenyl and octenyl, which is exemplified by naphthylethenyl, naphthylallyl, naphthylbutenyl, naphthylpentenyl, naphthylhexenyl and naphthyloctenyl; cycloalkylalkenyl is that wherein a cycloalkyl moiety is a cycloalkyl having 3 to 7 carbon atoms such as cyclopropyl, cyclobutyl, cyclopentyl, cyclohexyl and cycloheptyl and an alkenyl moiety is an alkenyl having 2 to 8 carbon atoms such as ethenyl, propenyl (e.g. allyl, 1-propenyl), butenyl (e.g. 1-butenyl, 2-butenyl, 3-butenyl), 3,3-dimethylallyl, pentenyl, hexenyl and octenyl, which is exemplified by cyclopropylethenyl, cyclobutylethenyl, cyclopentylethenyl, cyclohexylethenyl and cycloheptylethenyl; heteroaryl is a 5- or 6-membered ring having at least one of oxygen, sulfur and optionally substituted nitrogen atom (substituent being exemplified by alkyl, phenylalkyl and acyl), such as furan, thiophene, imidazole, pyrazole, tetrazole, pyridine, pyrimidine, benzofuran, benzothiophene, benzimidazole and quinoline; heteroarylalkyl is that wherein a heteroaryl moiety is a 5- or 6-membered ring having at least one of oxygen, sulfur and optionally substituted nitrogen atom (substituent being exemplified by alkyl, phenylalkyl and acyl), such as furan, thiophene, imidazole, pyrazole, tetrazole, pyridine, pyrimidine, benzofuran, benzothiophene, benzimidazole and quinoline and an alkyl moiety is a straight- or branched chain alkyl having 1 to 6 carbon atoms such as methyl, ethyl, propyl, isopropyl, butyl, pentyl and hexyl, which is exemplified by furfuryl, thienylmethyl, imidazolylmethyl, pyrazolylmethyl, tetrazolylmethyl, pyridylmethyl, pyrimidinylmethyl, benzofuranylmethyl, benzothienylmethyl, benzimidazolylmethyl, quinolylmethyl, furylethyl, thienylethyl, imidazolylethyl, pyrazolylethyl, pyridylethyl, pyrimidinylethyl, benzimidazolylethyl and quinolylethyl; phenylalkoxy is that wherein an alkoxy moiety is a straight- or branched chain alkoxy having 1 to 6 carbon atoms such as methoxy, ethoxy, propoxy, isopropoxy, butoxy, pentyloxy and hexyloxy, which is exemplified by benzyloxy, phenylethoxy, phenylpropoxy, 1-phenylethoxy, phenylbutoxy, phenylpentyloxy and phenylhexyloxy; heteroarylalkoxy is that wherein a heteroaryl moiety is a 5- or 6-membered ring having at least one of oxygen, sulfur and optionally substituted nitrogen atom (substituent being exemplified by alkyl, phenylalkyl and acyl) such as furan, thiophene, imidazole, pyrazole, tetrazole, pyridine, pyrimidine, benzofuran, benzothiophene, benzimidazole and quinoline and an alkoxy moiety is a straight- or branched chain alkoxy having 1 to 6 carbon atoms such as methoxy, ethoxy, propoxy, isopropoxy, butoxy, pentyloxy and hexyloxy, which is exemplified by furfuryloxy, thienylmethoxy, imidazolylmethoxy, pyrazolylmethoxy, tetrazolylmethoxy, pyridylmethoxy, pyrimidinylmethoxy, benzofuranylmethoxy, benzothienylmethoxy, benzimidazolylmethoxy, quinolylmethoxy, furylethoxy, thienylethoxy, imidazolylethoxy, pyrazolylethoxy, pyridylethoxy, pyrimidinylethoxy, benzimidazolylethoxy and quinolylethoxy; heteroaryloxy is that wherein a heteroaryl moiety is a 5- or 6-membered ring having at least one of oxygen, sulfur and optionally substituted nitrogen atom (substituent being exemplified by alkyl, phenylalkyl and acyl) such as furan, thiophene, imidazole, pyrazole, tetrazole, pyridine, pyrimidine, benzofuran, benzothiophene, benzimidazole and quinoline, which is exemplified by furyloxy, thienyloxy, imidazolyloxy, pyrazolyloxy, tetrazolyloxy, pyridyloxy, pyrimidinyloxy, benzofuranyloxy, benzothienyloxy, benzimidazolyloxy and quinolyloxy; heterocyclic aromatic ring is the same as the heteroaryl as mentioned above; heterocyclic ring formed by bonding with adjacent nitrogen atom is a 5- to 7-membered ring further having, as hetero atom(s), oxygen, sulfur and/or optionally substituted nitrogen atom (substituent being exemplified by alkyl, phenylalkyl and acyl) such as pyrrolidine, piperidine, piperazine, morpholine, homopiperazine, 4-ethylpiperazine, 4-(2-hydroxyethyl)piperazine, 2-aminomethylmorpholine and 2-hydroxymethylmorpholine; acyl is exemplified by alkanoyl having 2 to 6 carbon atoms (e.g. acetyl, propionyl, butyryl, pivaloyl and valeryl), benzoyl, substituted benzoyl (substituent being exemplified by halogen, alkyl, alkoxy and nitro), alkoxycarbonyl (e.g. methoxycarbonyl, ethoxycarbonyl, propoxycarbonyl, isopropoxycarbonyl, butoxycarbonyl, t-butoxycarbonyl) and phenylalkoxycarbonyl (e.g. benzyloxycarbonyl, phenylethoxycarbonyl, phenylpropoxycarbonyl, 1-phenylethoxycarbonyl, phenylbutoxycarbonyl); acyloxy is that wherein an acyl moiety is an alkanoyl having 2 to 6 carbon atoms (e.g. acetyl, propionyl, butyryl, pivaloyl, valeryl), benzoyl, substituted benzoyl (substituent being exemplified by halogen, alkyl, alkoxy and nitro), alkoxycarbonyl (e.g. methoxycarbonyl, ethoxycarbonyl, propoxycarbonyl, isopropoxycarbonyl, butoxycarbonyl, t-butoxycarbonyl) or phenylalkoxycarbonyl (e.g. benzyloxycarbonyl, phenylethoxycarbonyl, phenylpropoxycarbonyl, 1-phenylethoxycarbonyl, phenylbutoxycarbonyl), which is exemplified by acetoxy, propionyloxy, butyryloxy, pivaloyloxy, valeryloxy, benzoyloxy, substituted benzoyloxy (substituent being exemplified by halogen, alkyl, alkoxy and nitro), methoxycarbonyloxy, ethoxycarbonyloxy, propoxycarbonyloxy, isopropoxycarbonyloxy, butoxycarbonyloxy, t-butoxycarbonyloxy, benzyloxycarbonyloxy, phenylethoxycarbonyloxy, phenylpropoxycarbonyloxy, 1-phenylethoxycarbonyloxy and phenylbutoxycarbonyloxy; and straight- or branched chain alkylene is a straight- or branched chain alkylene having 1 to 8 carbon atoms, such as methylene, ethylene, methylmethylene, trimethylene, propylene, tetramethylene, pentamethylene, hexamethylene, octamethylene or 2-ethylhexamethylene.

The above-mentioned alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$,$-NHSO_2R^5$, $-CONR^6R^7$, $-NR^6R^7$, $-OCONR^6R^7$, $-OCSNR^6R^7$, $-SO_2NR^6R^7$ and-$COOR^8$ (wherein $R^5$, $R^6$, $R^7$, $R^8$ and m are as defined above).

As noted in the above, the substituents for the optionally substituted nitrogen atom are exemplified by alkyl, phenylalkyl and acyl and the substituents for substituted benzoyl and substituted benzoyloxy are exemplified by halogen, alkyl, alkoxy and nitro.

In all cases described in the foregoing, halogen as a substituent means chlorine, bromine, fluorine or iodine; alkyl as a substituent means a straight- or branched chain alkyl having 1 to 10 carbon atoms; alkoxy as a substituent means a straight- or branched chain alkoxy having 1 to 10 carbon atoms; phenylalkyl as a substituent means that wherein an alkyl moiety is a straight- or branched chain alkyl having 1 to 6 carbon atoms; acyl as a substituent means an alkanoyl having 2 to 6 carbon atoms, benzoyl, substituted benzoyl, alkoxycarbonyl or phenylalkoxycarbonyl; and acyloxy as a substituent means that wherein an acyl moiety is an alkanoyl having 2 to 6 carbon atoms, benzoyl, substituted benzoyl, alkoxycarbonyl or phenylalkoxycarbonyl.

When the compound of the present invention has an asymmetric carbon, the present invention encompasses racemates, diastereomers and optical isomers.

The pharmaceutically acceptable salts of the compound of the present invention include acid addition salts such as hydrochloride, hydrobromide, phosphate, sulfate, citrate, lactate, maleate, fumarate and tartrate. When the compound has a carboxyl group (when $R^8$ in $-COOR^8$ is hydrogen), metal salts such as sodium salt, potassium salt, calcium salt and aluminium salt, and salts with amino acid such as lysine and ornithine are encompassed. In addition, solvates such as hydrates (1/2 hydrate, monohydrate, 3/2 hydrate etc.) are also encompassed.

The compound of the formula (I) of the present invention is produced by a method known per se. In the present specification, production methods for the compounds of the formulas (II) through (IV) which are encompassed by the formula (I) are respectively explained in the following.

For example, compounds of the formula (II) can be produced by the following method. That is, a compound of the formula

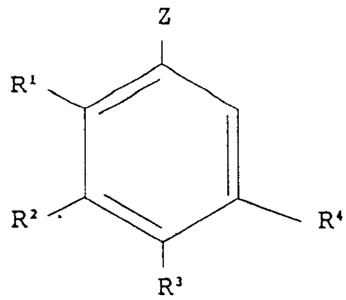

(V)

wherein Z is hydroxyl or methoxy and other symbols are as defined above [which can be synthesized, for example, by the method described in J. Chem. Soc., p. 440 (1927), hereinafter referred to as Compound (V)] and a compound of the formula

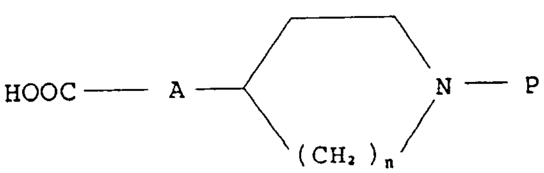

(VI)

wherein P is the same as R or a protecting group such as acetyl, benzoyl, t-butoxycarbonyl or benzyloxycarbonyl and other symbols are as defined above [which can be synthesized, for example, by the method described in Journal of the American Chemical Society, Vol. 65, pp. 2460-2465 (1943) and GB Patent No. 2000136, hereinafter referred to as Compound (VI)] or its reactive derivative are subjected to Friedel-Crafts' reaction to give a compound of the formula

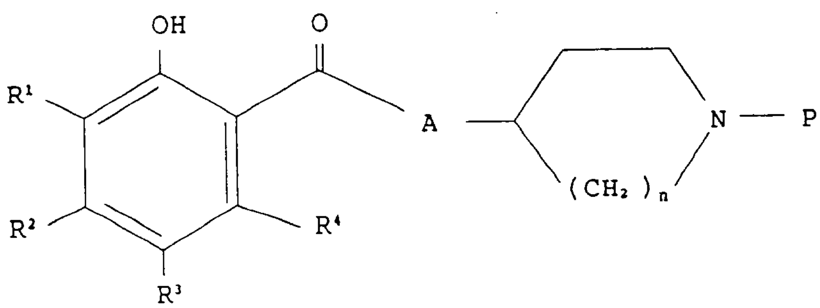

(VII)

wherein each symbol is as defined above [hereinafter referred to as Compound (VII)]; the Compound (VII) is condensed with hydroxylamine to convert same to an oxime derivative of the formula

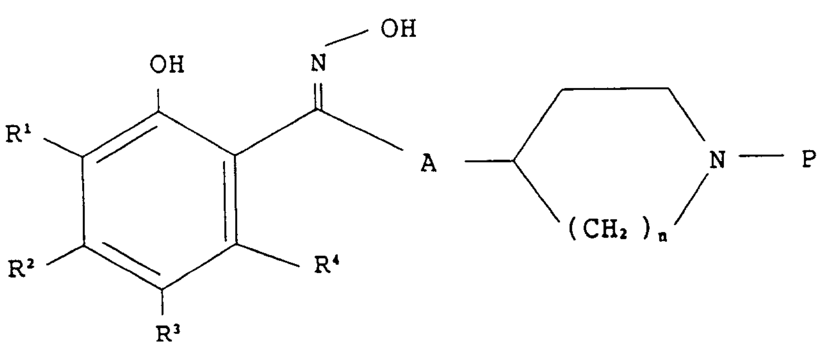

(VIII)

wherein each symbol is as defined above [hereinafter referred to as Compound (VIII)].

Then, the Compound (VIII) is reacted with a compound of the formula

W-COOH (IX)

wherein W is a lower alkyl which may be substituted by halogen, or phenyl which may be substituted by nitro [hereinafter referred to as Compound (IX)] or its reactive derivative for acylation to give a compound of the formula

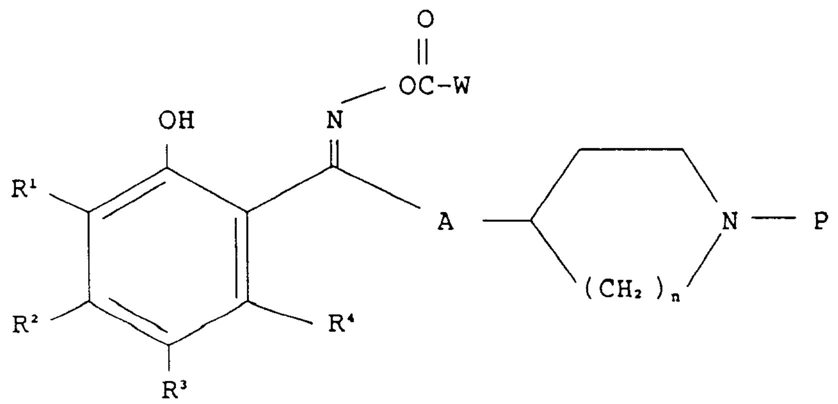

(X)

wherein each symbol is as defined above [hereinafter referred to as Compound (X)]; the Compound (X) is subjected to a ring-closure reaction to produce a compound of the formula

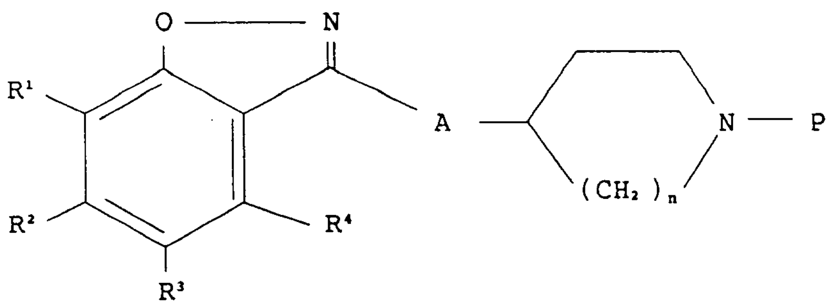

(XI)

wherein each symbol is as defined above [hereinafter referred to as Compound (XI)]. When P is an amine-protecting group such as acetyl, benzoyl, t-butoxycarbonyl or benzyloxycarbonyl, the Compound (XI) is subjected to a deprotection reaction to give a compound of the formula

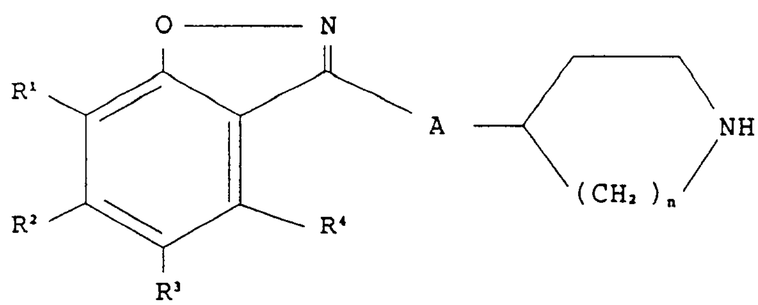

(XII)

wherein each symbol is as defined above [hereinafter referred to as Compound (XII)]; the Compound (XII) is reacted with a compound of the formula

X-R' (XIII)

wherein X is a leaving group such as halogen, 4-methylphenylsulfonyloxy, methylsulfonyloxy or trifluoromethylsulfonyloxy and R' is a group for R except hydrogen [hereinafter referred to as Compound

(XIII)] to give a Compound (II) wherein R is other than hydrogen.

The Compound (XI) can be also produced by the following synthetic method. That is, a compound of the formula

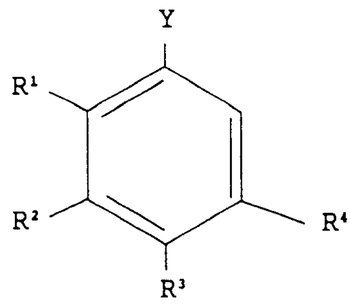

(XIV)

wherein Y is a halogen atom such as fluorine or chlorine and other symbols are as defined above [which can be synthesized, for example, by the method described in Journal of the American Chemical Society, Vol. 71, p. 411 (1949), hereinafter referred to as Compound (XIV)]; the Compound (VI) are subjected to Friedel-Crafts' reaction to give a compound of the formula

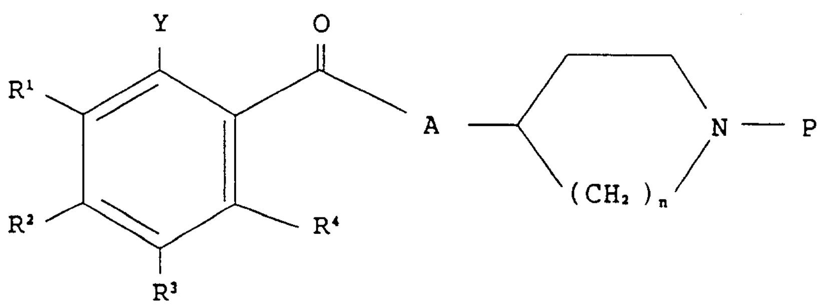

(XV)

wherein each symbol is as defined above [hereinafter referred to as Compound (XV)] and the Compound (XV) is condensed with hydroxylamine to give a compound of the formula

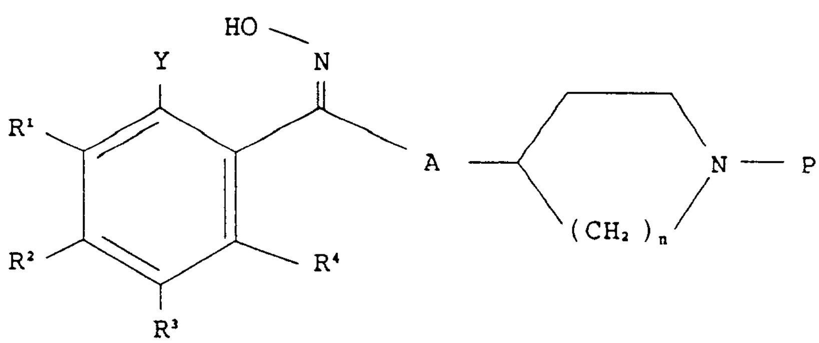

(XVI)

wherein each symbol is as defined above [hereinafter referred to as Compound (XVI)]. Then, the Compound (XVI) is subjected to a ring-closure reaction to give a Compound (XI).

The Friedel-Crafts' reaction between the Compound (V) or the Compound (XIV) and the Compound (VI) (reactive derivatives such as acid chloride, acid anhydride, mixed acid anhydride and activated ester are preferable and acid chloride is particularly preferable) proceeds without or in a solvent which does not interfere with the reaction, such as 1,2-dichloroethane, nitrobenzene, dichloromethane or carbon tetrachloride using, as a catalyst, a Lewis acid such as aluminium chloride, ferric chloride or stannic chloride, or an acid such as sulfuric acid or polyphosphoric acid under cooling, at room temperature or under heating.

The conversion of the Compound (VII) and the Compound (XV) to oxime derivatives proceeds in a solvent inert to the reaction, using hydroxylamine hydrochloride and, where necessary, a buffer or triethylamine, sodium acetate, potassium carbonate, sodium carbonate or the like under cooing, at room temperature or under heating.

The acylation of the Compound (VIII) proceeds according to the method given under (1) - (4) below.

(1) When the Compound (IX) is a free carboxylic acid, the reaction proceeds in an inert solvent in the presence of a condensing agent such as dicyclohexylcarbodiimide, titanium tetrachloride, phosphorus halide (e.g. phosphorus trichloride, phosphorus oxychloride), diethyl chlorophosphite, o-phenylene chlorophosphite or ethyl dichlorophosphite under cooling, at room temperature or under heating.

(2) When an acid halide such as acid chloride or acid bromide is used as a reactive derivative of the Compound (IX), the reaction is carried out in an inert solvent in the presence of a tertiary base such as triethylamine, pyridine or N,N-dimethylaniline under cooling or at room temperature; or carried out in water in the presence of an alkali such as sodium hydroxide or potassium hydroxide under cooling or at room temperature.

(3) When a symmetrical acid anhydride or a mixed acid anhydride (e.g. mixed acid anhydride of alkyl carbonic acid, mixed acid anhydride of alkyl phosphoric acid, mixed acid anhydride of alkyl phosphorous acid, mixed acid anhydride of sulfuric acid) is used as a reactive derivative of the Compound (IX), the reaction proceeds in an inert solvent using, where necessary, a tertiary base such as triethylemine, pyridine or N,N-dimethylaniline under cooling, at room temperature or under heating.

(4) When an activated amide such as acid imidazolide, acid pyrrolidide or 2,4-dimethylpyrazolide is used as a reactive derivative of the Compound (IX), the reaction proceeds in an inert solvent at room temperature or under heating.

The ring-closure reaction of the Compound (X) and the Compound (XVI) proceeds in a solvent inert to the reaction using a base such as sodium hydride, potassium t-butoxide, sodium ethoxide, sodium hydroxide or potassium hydroxide at room temperature or under heating.

The reaction conditions for the deprotection of the Compound (XI) are appropriately selected according to the kind of the protecting group P. For example, when the protecting group P is an amide type protecting group such as acetyl or benzoyl, deprotection is performed by hydrolysis with an acid or a base.

The reaction between the Compound (XII) and the Compound (XIII) proceeds in an inert solvent using, where necessary, an organic base such as triethylamine, dimethylaminopyridine or pyridine, a metal alcoholate such as sodium ethylate or sodium methylate, or an inorganic base such as potassium carbonate, sodium carbonate, sodium hydrogencarbonate, sodium hydroxide, potassium hydroxide, potassium hydride or sodium hydride at room temperature or under heating.

Compounds of the formula (III) can be produced, for example, by the following method. That is, a compound of the formula

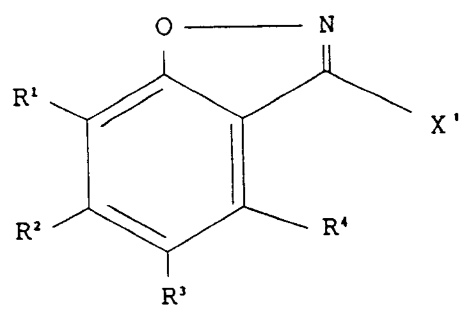

(XVII)

wherein X' is a halogen such as chlorine and other symbols are as defined above [hereinafter referred to as Compound (XVII)] is reacted with a compound of the formula

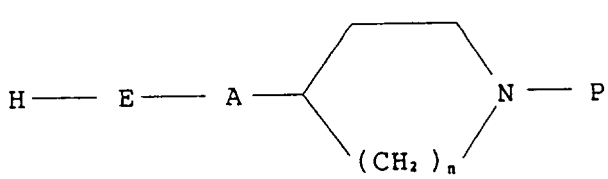

(XVIII)

wherein each symbol is as defined above [which can be synthesized, for example, by the method described in Japanese Patent Unexamined Publication No. 130973/1980, hereinafter referred to as Compound (XVIII)] to produce a novel compound of the formula

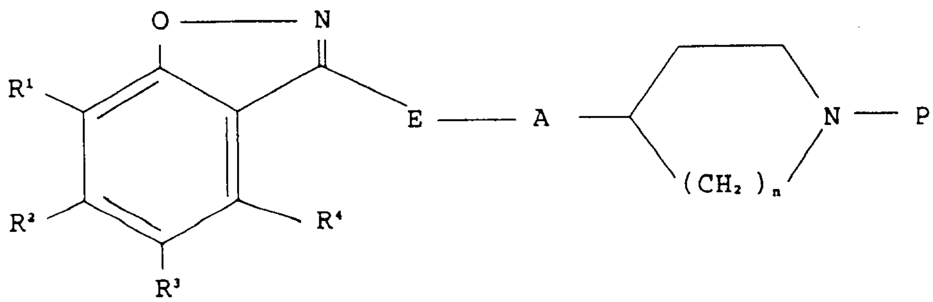

(XIX)

wherein each symbol is as defined above [hereinafter referred to as Compound (XIX)]. When P in the Compound (XIX) is an amine-protecting group such as acetyl, benzoyl, tert-butoxycarbonyl or benzyloxycarbonyl, the Compound (XIX) is subjected to a deprotection reaction to give a novel compound of the formula

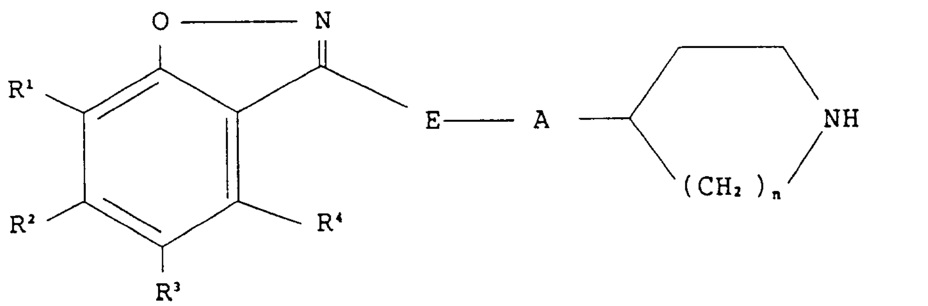

(XX)

wherein each symbol is as defined above [hereinafter referred to as Compound (XX)]; the Compound (XX) is reacted with the compound (XIII) to give a Compound (III) wherein R is other than hydrogen [when E in (XX) is oxygen].

The Compound (XIX) wherein E is oxygen can be also obtained by the method shown in the following. That is, a compound of the formula

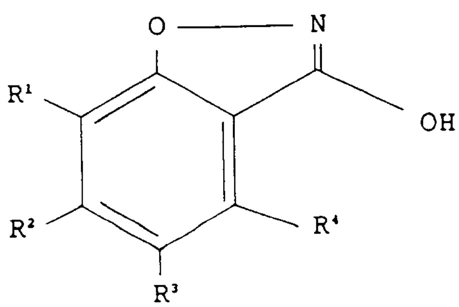

(XXI)

wherein each symbol is as defined above [hereinafter referred to as Compound (XXI)] is reacted with a compound of the formula

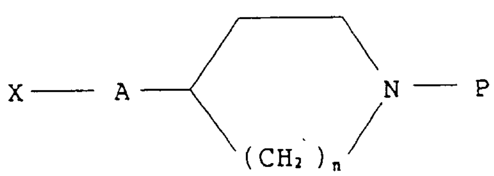

(XXII)

wherein each symbol is as defined above [which can be synthesized, for example, by the method described in Chemical Pharmaceutical Bulletin, Vol. 34, p. 3747 (1986), hereinafter referred to as Compound (XXII)] to produce a Compound (XIX) wherein E is oxygen.

The reactions between the Compounds (XVII) and (XVIII), the Compounds (XX) and (XIII), and the Compounds (XXI) and (XXII) proceed in an inert solvent using, where necessary, an organic base such as

triethylamine, dimethylaminopyridine or pyridine, a metal alcoholate such as sodium ethylate or sodium methylate, or an inorganic base such as potassium carbonate, sodium carbonate, sodium hydrogencarbonate, sodium hydroxide, potassium hydroxide or sodium hydride at room temperature or under heating.

The reaction conditions for the deprotection of the Compound (XIX) are appropriately selected according to the kind of the protecting group P. For example, when the protecting group P is an amide type protecting group such as acetyl or benzoyl, deprotection is performed by hydrolysis with an acid or a base. When the protecting group P is t-butoxycarbonyl or benzyloxycarbonyl, deprotection is performed with a hydrogen bromide-acetic acid solution.

Compounds of the formula (IV) of the present invention can be produced, for example, by the following method. That is, a compound of the formula

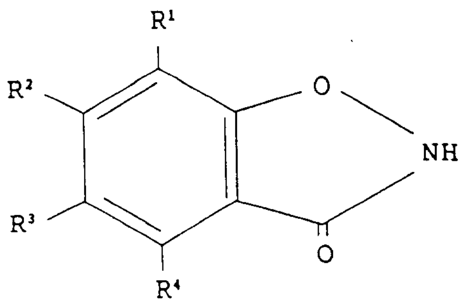

(XXIII)

wherein each symbol is as defined above [hereinafter referred to as Compound (XXIII)] is reacted with the Compound (XXII) to give a novel compound of the formula

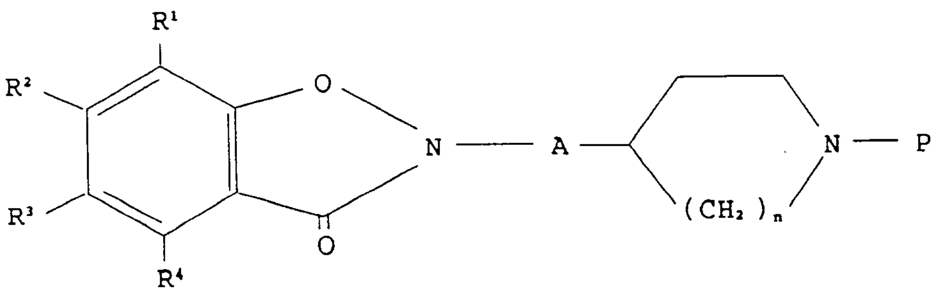

(XXIV)

wherein each symbol is as defined above [hereinafter referred to as Compound (XXIV)]. When P in the Compound (XXIV) is an amine-protecting group such as acetyl, benzoyl, t-butoxycarbonyl or benzyloxycarbonyl, the Compound (XXIV) is subjected to deprotection to give a novel compound of the formula

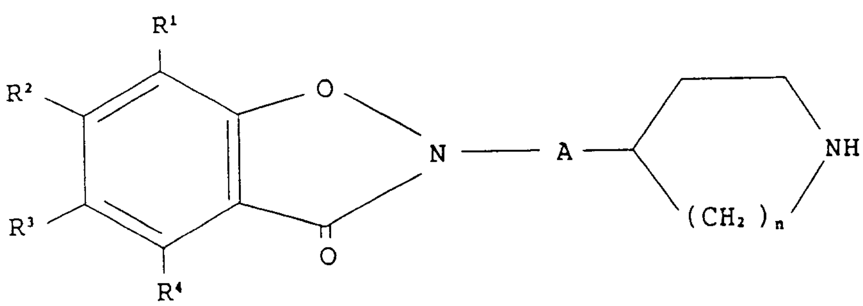

(XXV)

wherein each symbol is as defined above [hereinafter referred to as Compound (XXV)]. The Compound (XXV) is reacted with the Compound (XIII) to give a compound (IV) wherein R is other than hydrogen.

The reactions between the Compounds (XXIII) and (XXII), and the Compounds (XXV) and (XIII) proceed in an inert solvent using, where necessary, an organic base such as triethylamine, dimethylaminopyridine or pyridine, a metal alcoholate such as sodium ethylate or sodium methylate, or an inorganic base such as potassium carbonate, sodium carbonate, sodium hydrogencarbonate, sodium hydroxide, potassium hydroxide or sodium hydroxide at room temperature or under heating.

The reaction conditions for the deprotection of the Compound (XXIV) are appropriately selected according to the kind of the protecting group P. For example, when the protecting group P is an amide type protecting group such as acetyl or benzoyl, deprotection is performed by hydrolysis with an acid or a base. When the protecting group P is t-butoxycarbonyl or benzyloxycarbonyl, deprotection is performed with a hydrogen bromide-acetic acid solution.

The $R^1$ to $R^4$ of the Compound (I) of the present invention can be converted to other substituents by the method known in the organic synthetic chemistry such as acylation or sulfonylation of hydroxyl group or amino group, or esterification or amidation of carboxylic acid group.

The Compounds (XXI) and (XXIII) which are the starting compounds in the present invention are known compounds and can be synthesized by the method described in Chem. Ber., Vol. 100, p. 954 (1967).

Of the starting compounds (XVII), that wherein X' is chlorine is also known and can be synthesized by the method described in Journal of the Medicinal Chemistry, Vol. 29, p. 359 (1986).

When the compound of the present invention has an asymmetric center, it is generally produced as racemates which can be optically resolved into optical isomers by conventional methods such as fractional recrystallization and chromatography. In addition, an optical isomer can be produced from an optically active starting compound. When the compound has at least two asymmetric central nervous systems, it is generally produced as respective diastereomers or mixtures thereof. The respective diastereomers can be separated by a method such as fractional recrystallization or chromatography.

The compounds of the present invention which are obtainable as described above can be converted to acid addition salts such as hydrochloride, hydrobromide, phosphate, sulfate, citrate, lactate, maleate, fumarate and tartrate by a method known per se. When the compound has a carboxyl group, it can be converted to a metal salt such as sodium salt, potassium salt, calcium salt or aluminium salt, or a salt with amino acid such as lysine or ornithine.

When the Compound (I) of the present invention or a pharmaceutically acceptable salt thereof is used as a pharmaceutical, it is mixed with carriers, exipients, diluents, solubilizers and so on as appropriate and prepared into tablets, capsules, powders, injections, transfusions etc. While the dose varies depending on age, sex, symptom etc. of patients, it is generally administered to an adult at 0.1 - 300 mg, preferably 1 - 100 mg daily in a single to several times divided doses.

The effects of the compounds of the present invention are described in detail by means of the following Experimental Examples.

Experimental Example 1

The experiment was carried out according to the method of Ellmann et al. [Biochem. Pharmacol. Vol. 7, pp. 88-95 (1961)].

An enzyme solution was a homogenized solution of forebrain from male Wistar rat in 0.1M phosphate buffer (pH 8.0). Acetylthiocholine was used as a substrate for acetylcholinesterase (AChE) and the density of thionitrobenzoate (yellow) produced by the following reaction was measured with a spectrophotometer ($OD_{412}$).

$$\text{Acetylthiocholine} \xrightarrow{\text{AChE}} \text{thiocholine} + \text{acetate}$$

Thiocholine + dithiobisnitrobenzoate → thionitrobenzoate

Test compounds were dissolved in dimethylsulfoxide and diluted with 0.1M phosphate buffer (pH 8.0).

Inhibitory effects of the compounds of the present invention on acetylcholinesterase, which were determined as shown in the above are summarized in Table 1.

Table 1

| Example compound No. and Comparison compound | Acetylcholinesterase inhibition $IC_{50}$ (nM) |
|---|---|
| 19 | 0.38 |
| 23 | 0.34 |
| 252 | 0.73 |
| THA | 36 |

Experimental Example 2

Inhibitory effects of the compounds on acetylcholinesterase and butyrylcholinesterase were determined according to the method of Ellmann et al. as described in Experimental Example 1, using acetylcholinesterase (AChE) derived from human and butyrylcholinesterase (BChE) derived from human as enzymes and acetylthiocholine and butyrylthiocholine as substrates, respectively. The concentration of test compounds which was necessary for inhibiting butyrylcholinesterase by 50% ($IC_{50}$) was divided by the $IC_{50}$ for acetylcholinesterase and the obtained value was used as an index to show selectivity to acetylcholinesterase.

The selectivity of the compounds of the present invention to acetylcholinesterase as determined by the method as described above is shown in Table 2.

Table 2

| Example compound No. and Comparison compound | $IC_{50}$ (nM) | | Selectivity $IC_{50}$ (BChE)/$IC_{50}$ (AChE) |
|---|---|---|---|
| | AChE | BChE | |
| 6 | 2.1 | 9,500 | 4,500 |
| 11 | 0.5 | >10,000 | >20,000 |
| 19 | 1.8 | 7,500 | 4,200 |
| 41 | 4.6 | 17,000 | 3,700 |
| 198 | 0.94 | 3,600 | 3,800 |
| THA | 100 | 9.6 | 0.1 |

As shown in the results of Table 1 and Table 2, the compounds of the present invention were found to potently and center-selectively inhibit acetylcholinesterase.

Experimental Example 3 : Sigma receptor binding test

Male Wistar rats weighing 250-300 g were decapitated and the brains were removed. Cerebral cortex was dissected on ice, and the brain tissues were homogenized in 20 volumes of Tris-HCl buffer (pH 8.0, 25°C) containing 320 mM sucrose. The homogenate was centrifuged at 1000 × g for 10 minutes. The obtained supernatant was centrifuged at 31,000 × g for 15 minutes and the pellets obtained were suspended in the same Tris-HCl buffer, followed by incubation at 37°C for 10 minutes. Centrifugation was repeated twice and the final pellets were suspended in 50 mM Tris-HCl buffer (pH 8.0, 25°C) and used as membrane fractions.

A binding inhibitory test was conducted by adding [$^3$H]-1,3-di-o-tolylguanidine ($^3$H-DTG, 39.4 Ci/mmol, New England Nuclear, final concentration 2.5 nM) and respective test compounds (5 different concentrations) to the membrane fractions (0.9 ml, 0.5-0.8 mg) and then the above-mentioned buffer to the final volume of 1 ml. The mixtures were incubated at 25°C for 60 minutes. The reaction mixture was suction-filtered through Whatman GF/B filters which had been treated previously with 0.1% polyethyleneimine and washed three times with 3 ml of 10 mM Tris-HCl buffer (pH 7.7, 25°C). Then, the $\beta$ rays of the bound $^3$H-DTG on the filter were measured by a liquid scintillation counter.

$IC_{50}$ values were determined graphically and Ki values were calculated by the numerical equation

$$Ki = IC_{50}/[1 + (L/Kd)]$$

wherein L is a concentration of radioligand and Kd (26.3 nM) is a dissociation constant between tritiated ligand and the receptor.

The Ki values of the compounds of the present invention for sigma receptor as obtained by the method as described above are shown in Table 3.

Table 3

| Example compound No. | Sigma receptor binding assay $^3$H-DTG (Ki:nM) |
|---|---|
| 160 | 0.23 |
| 164 | 0.37 |
| 227 | 0.55 |
| 231 | 0.42 |

Experimental Example 4 : Acute toxicity

The acute toxicity values ($LD_{50}$) of the compounds of the present invention 6 and 41 after oral administration to rats were not less than 50 mg/kg.

From the foregoing Experimental Examples and various pharmacological tests, it has been confirmed that the compounds of the present invention inhibit acetylcholinesterase in the central nervous system potently and selectively and are useful for the prophylaxis, improvement and treatment of Alzheimer-type senile dementia, cerebral hemorrhage, cerebrovascular disorders caused by head trauma, lowered volition, emotional disturbance, amnesia, allophasis, disorientation and the like.

In addition, the compounds of the present invention exhibit high and long-lasting affinity for the sigma receptor and are useful as agents for the central nervous system for the prophylaxis improvement and treatment of various diseases such as psychosis, depression, anxiety and the like.

While the present invention is described in detail in the following by illustrating Reference Examples and Examples, the invention is not limited thereto.

Reference Example 1

1-Acetyl-4-piperidone (51.4 g) and β-carboxyethyltriphenyl phosphonium chloride (134.9 g) were dissolved in a mixed solvent of dimethylsulfoxide (500 ml) and tetrahydrofuran (500 ml) and 60% sodium hydride (32.1 g) was added thereto over 50 minutes while adjusting the temperature of the reaction mixture to 0°C - 1°C. The mixture was stirred for 5 more hours while keeping the temperature at 0°C and then stirred at room temperature overnight. The reaction mixture was concentrated and water (1000 ml) was added thereto. The precipitated crystals were filtered off and the filtrate was extracted with benzene. The aqueous layer was concentrated and water (200 ml) was added thereto. Conc. hydrochloric acid was added thereto under ice-cooling to make the reaction mixture acidic. The precipitated crude crystals were collected by filtration and recrystallized from isopropyl alcohol to give 38.5 g of 3-(1-acetyl-4-piperidylidene)propionic acid, melting point 115°C - 119°C.

Reference Example 2

3-(1-Acetyl-4-piperidylidene)propionic acid (18.2 g) was dissolved in methanol (500 ml) and Raney-nickel was added thereto to carry out catalytic hydrogenation at atmospheric pressure. After the completion of the reaction, the catalyst was collected by filtration and the filtrate was concentrated. Ethyl acetate was added thereto and the precipitated crystals were collected by filtration to give 16.6 g of 3-(1-acetyl-4-piperidinyl)propionic acid, melting point 116°C - 120°C.

Reference Example 3

3-(1-Acetyl-4-piperidyl)propionic acid (16.6 g) was suspended in dichloroethane (100 ml) and thionyl chloride (7.8 ml) was added thereto. After being stirred at room temperature for 2 hours, the reaction

mixture was concentrated to give 20.8 g of 3-(1-acetyl-4-piperidyl)propionic chloride, melting point 108 - 109°C.

Reference Example 4

m-Difluorobenzene (7.8 g) was dissolved in dichloromethane (16 ml) and aluminium chloride (15 g) was added thereto. Then, a solution of 3-(1-acetyl-4-piperidyl)propionic chloride in dichloromethane (120 ml) was added thereto over 40 minutes with stirring. The reaction mixture was stirred at room temperature for 2 hours and refluxed for 4 hours. The reaction mixture was poured into ice water, extracted with chloroform and purified by silica gel chromatography to give 11.5 g of 2',4'-difluoro-3-(1-acetyl-4-piperidyl)-propiophenone, melting point 75 - 82°C.

Reference Example 5

2',4'-Difluoro-3-(1-acetyl-4-piperidyl)propiophenone (11.5 g) was suspended in 6N hydrochloric acid and refluxed under stirring for 4 hours. The reaction mixture was concentrated and isopropyl alcohol was added thereto. The precipitated crystals were collected by filtration to give 8.1 g of 2',4'-difluoro-3-(4-piperidyl)-propiophenone hydrochloride, melting point 194 - 198°C.

Reference Example 6

2',4'-Difluoro-3-(4-piperidyl)propiophenone hydrochloride (7.2 g) and calcium carbonate (8.6 g) were suspended in acetone (150 ml) and benzyl chloride (4.4 g) was added thereto. After being stirred at room temperature for 18 hours, the reaction mixture was concentrated, exracted with ethyl acetate, washed with water and concentrated to give 6.8 g of 2',4'-difluoro-3-(1-benzyl-4-piperidyl)propiophenone.

$^1$H-NMR($CDCl_3$)$\delta$ : 1.03-2.22(m,7H), 2.58-3.11(m,4H), 4.45(s,2H), 6.55-7.05(m,2H), 7.19(s,5H), 7.64-8.03-(m,1H)

Reference Example 7

2',4'-Difluoro-3-(1-benzyl-4-piperidyl)propiophenone (6.8 g), hydroxylamine hydrochloride (3.4 g) and triethylamine (6.0 g) were dissolved in methanol (200 ml) and the mixture was stirred at 50 - 60°C for 22 hours. The reaction mixture was concentrated, extracted with ethyl acetate and purified by silica gel chromatography to give 2.2 g of syn-2',4'-difluoro-3-(1-benzyl-4-piperidyl)propiophenone oxime, melting point 100 - 104°C.

Example 1

Syn-2',4'-difluoro-3-(1-benzyl-4-piperidyl)propiophenone oxime (2.4 g) was dissolved in ethanol (45 ml) and 60% sodium hydride (1.0 g) was added thereto. After being stirred at 60°C for 4 hours, the reaction mixture was concentrated and the residue obtained was extracted with chloroform and washed with water. After being dried over magnesium sulfate, it was concentrated under reduced pressure and the residue obtained was purified by silica gel chromatography to give 1.2 g of 3-(2-(1-benzyl-4-piperidyl)ethyl)-6-fluoro-1,2-benzoisoxazole as an oily substance. Maleic acid (0.45 g) and ethyl acetate were added to the oily substance. The precipitated crystals were collected by filtration. The obtained crystals were recrystallized from isopropyl alcohol to give 1.0 g of 3-(2-(1-benzyl-4-piperidyl)ethyl)-6-fluoro-1,2-benzisoxazole maleate, melting point 153 - 154°C.

Reference Example 8

N-(3-Methoxyphenyl)benzamide (4.4 g) and 3-(1-acetyl-4-piperidyl)propionyl chloride (5.4 g) were dissolved in dichloroethane (120 ml) and aluminium chloride (11 g) was added thereto under ice-cooling. The reaction mixture was allowed to gradually become room temperature. After being stirred for 6 hours, the reaction mixture was poured into ice water, extracted with chloroform and purified by silica gel chromatography to give 5.0 g of 3-(1-acetyl-4-piperidyl)-4'-benzoylamino-2'-hydroxypropiophenone, melting point 179 - 181°C.

Reference Example 9

Methanol (230 ml) was added to 3-(1-acetyl-4-piperidyl)-4'-benzoylamino-2'-hydroxypropiophenone (5.3 g) and the mixture was refluxed under stirring for 30 minutes to dissolve crystals. After cooling to room temperature, hydroxylamine hydrochloride (2.3 g) and triethylamine (4.1 g) were added thereto and the mixture was stirred at 50 ° C for 17 hours. The reaction mixture was concentrated and water was added to allow precipitation of crystals. The crystals were collected by filtration and washed with water to give 4.5 g of 3-(1-acetyl-4-piperidyl)-4'-benzoylamino-2'-hydroxypropiophenone oxime, melting point 219 - 220 ° C.

Reference Example 10

3-(1-Acetyl-4-piperidyl)-4'-benzoylamino-2'-hydroxypropiophenone oxime (4.5 g) was dissolved in a mixed solvent of dimethylformamide (40 ml) and chloroform (100 ml) and acetic anhydride (1.5 g) was added thereto under ice-cooling. The reaction mixture was allowed to become room temperature and stirred overnight. The mixture was extracted with chloroform and washed with water. The extract was concentrated to give 5.1 g of 3-(1-acetyl-4-piperidyl)-4'-benzoylamino-2'-hydroxypropiophenone oxime acetate, melting point 170 - 173 ° C.

Reference Example 11

Sodium hydride (60%, 0.5 g) was added to dimethylformamide (60 ml) and a solution of 3-(1-acetyl-4-piperidyl)-4'-benzoylamino-2'-hydroxypropiophenone oxime acetate (5.1 g) in dimethylformamide (60 ml) was added dropwise thereto under ice-cooling over 5 minutes. The reaction mixture was allowed to become room temperature, stirred overnight and concentrated under reduced pressure. The residue was extracted with chloroform, washed with water, dried over magnesium sulfate and concentrated under reduced pressure. Ethyl acetate and isopropyl ether were added to the residue. The precipitated crystals were collected by filtration and washed with isopropyl ether to give 3.8 g of 3-(2-(1-acetyl-4-piperidyl)ethyl)-6-benzoylamino-1,2-benzoyloxazole, melting point 178 - 179 ° C.

Example 2

3-(2-(1-Acetyl-4-piperidyl)ethyl)-6-benzoylamino-1,2-benzisoxazole (3.8 g) was suspended in 3N hydrochloric acid (100 ml) and refluxed under stirring overnight. Ice-cooling of the obtained solution yielded crystals, which were collected by filtration. The filtrate was concentrated to give crystals. The crystals were washed with ether to give 3.0 g of 6-amino-3-(2-(4-piperidyl)ethyl)-1,2-benzisoxazole dihydrochloride, melting point more than 270 ° C.

$^1$H-NMR($D_2O$)$\delta$ : 1.42-1.52(m,2H), 1.64-1.75(m,1H), 1.87(q,2H), 2.03(d,2H), 2.97(t,2H), 3.13(t,2H), 3.44-(d,2H), 7.34(dd,1H), 7.60(d,1H), 7.94(d,1H)

Reference Example 12

3-(4-Piperidyl)propionic hydrochloride (39 g) and sodium acetate (33 g) were suspended in acetic acid (300 ml) and benzoylchloride (30 g) was added thereto under ice-cooling over 15 minutes. After being stirred at room temperature overnight, the reaction mixture was concentrated and extracted with ethyl acetate. The extract was dried and concentrated under reduced pressure to afford 61 g of 3-(1-benzoyl-4-piperidyl)propionic acid.

$^1$H-NMR($CDCl_3$)$\delta$ : 0.76-1.90(m,7H), 2.34(t,2H), 2.50-3.10(brs, 2H), 3.38-3.92(brs,1H), 4.36-4.83(brs,1H), 7.22-7.50(m,5H)

Reference Example 13

3-(1-Benzoyl-4-piperidyl)propionic acid (61 g) was suspended in dichloroethane (400 ml) and thionyl chloride (36 g) was added thereto. The reaction mixture was stirred at room temperature for 4 hours. The reaction mixture was concentrated to give 59 g of 3-(1-benzoyl-4-piperidyl)propionyl chloride. This acid chloride (12.0 g) and N-(3-methoxyphenyl)benzamide (9.2 g) were dissolved in dichloroethane (200 ml) and aluminium chloride (22.8 g) was added thereto under ice-cooling. After being stirred under ice-cooling for 43 hours, the reaction mixture was poured into cold water. Extraction with chloroform and purification by silica gel chromatography afforded 3.5 g of 3-(1-benzoyl-4-piperidyl) -4'-benzoylamino-2'-hydroxypropiophene.

$^1$H-NMR($CDCl_3$)δ : 2.68-2.87(brs,1H), 2.98(t,2H), 3.70-3.87(brs,1H), 4.65-4.82(brs,1H), 7.37(dd,1H), 7.75-(d,1H), 8.01(s,1H)

Reference Example 14

3-(1-Benzoyl-4-piperidyl)-4'-benzoylamino-2'-hydroxypropiophenone (3.5 g) and hydroxylamine hydrochloride (1.3 g) were suspended in methanol (35 ml) and triethylamine (2.3 g) was added thereto at 50 ° C. After being stirred at 50 ° C overnight, the reaction mixture was concentrated and water was added thereto. The precipitated crystals were collected by filtration and washed with water to give 3.2 g of 3-(1-benzoyl-4-piperidyl)-4'-benzoylamino-2'-hydroxypropiophenone oxime, melting point 250 - 251 ° C.

Reference Example 15

3-(1-Benzoyl-4-piperidyl)-4'-benzoylamino-2'-hydroxypropiophenone oxime (3.1 g) was dissolved in dimethylformamide (70 ml) and acetic anhydride (1.1 g) was added thereto under ice-cooling. The reaction mixture was allowed to become room temperature and stirred overnight, after which the reaction mixture was extracted with ethyl acetate and washed with water. The extract was concentrated. The precipitated crystals were collected by filtration and washed with isopropyl ether to give 3.5 g of 3-(1-benzoyl-4-piperidyl)-4'-benzoylamino-2'-hydroxypropiophenone oxime acetate, melting point 99 - 103 ° C.

Reference Example 16

Sodium hydride (60%, 0.13 g) was added to dimethylformamide (15 ml) and a solution of 3-(1-benzoyl-4-piperidyl)-4'-benzoylamino-2'-hydroxypropiophenone oxime acetate (1.5 g) in dimethylformamide (15 ml) was added dropwise thereto under ice-cooling. The reaction mixture was allowed to become room temperature. After being stirred overnight, the mixture was concentrated under reduced pressure. The precipitated crystals were collected by filtration and washed with water and ethyl acetate to give 1.1 g of 3-(2-(1-benzoyl-4-piperidyl)ethyl)-6-benzoylamino-1,2-benzoyloxazole, melting point 188 - 190 ° C.

Example 3

3-(2-(1-Benzoyl-4-piperidyl)ethyl)-6-benzoylamino-1,2-benzisoxazole (0.5 g) was suspended in conc. hydrochloric acid (5 ml) and acetic acid (5 ml) and refluxed under stirring for 10 hours. The obtained solution was concentrated under reduced pressure and water (30 ml) was added thereto. The precipitated crystals were filtered off and the filtrate was concentrated to give 0.25 g of 6-amino-3-(2-(4-piperidyl)ethyl)-1,2-benzisoxazole dihydrochloride, melting point not less than 270 ° C.

$^1$H-NMR($D_2O$)δ : 1.42-1.53(m,2H), 1.64-1.76(m,1H), 1.87(q,2H), 2.04(d,2H), 2.97(t,2H), 3.13(t,2H), 3.45-(d,2H), 7.34(dd,1H), 7.60(d,1H), 7.94(d,1H)

Example 4

6-Amino-3-(2-(4-piperidyl)ethyl)-1,2-benzisoxazole dihydrochloride (3.0 g) and potassium carbonate (7.8 g) were suspended in dimethylformamide (30 ml). After stirring at room temperature for 2 hours, benzyl bromide (1.9 g) was added thereto and the mixture was stirred at room temperature for 1 hour. The reaction mixture was concentrated and extracted with ethyl acetate. The mixture was washed with water, dried over magnesium sulfate and concentrated under reduced pressure to give 3.4 g of 6-amino-3-(2-(1-benzyl-4-piperidyl)ethyl)-1,2-benzisoxazole.

$^1$H-NMR($CDCl_3$)δ : 1.38(brs,2H), 1.72-1.80(m,4H), 2.00(brs,1H), 2.84-2.96(m,4H), 3.56(s,2H), 4.05(s,2H), 6.61(d,1H), 6.71(d,1H), 7.29-7.37(m,6H)

Example 5

6-Amino-3-(2-(1-benzyl-4-piperidyl)ethyl)-1,2-benzisoxazole (1.6 g) was dissolved in chloroform (15 ml) and benzoic anhydride (1.6 g) was added thereto. The mixture was stirred at room temperature for 10 hours. After completion of the reaction, aqueous sodium bicarbonate was added thereto and the mixture was extracted with chloroform, washed with water, dried and concentrated under reduced pressure. The obtained crystals were recrystallized from ethyl acetate to give 1.5 g of 6-benzoylamino-3-(2-(1-benzyl-4-piperidyl)ethyl)-1,2-benzisoxazole, melting point 160 - 162 ° C.

Example 6

6-Amino-3-(2-(1-benzyl-4-piperidyl)ethyl)-1,2-benzisoxazole (0.5 g) was dissolved in chloroform (5 ml) and acetic anhydride (5 ml) was added thereto. The mixture was stirred at room temperature overnight. After the completion of the reaction, aqueous sodium bicarbonate was added thereto and the mixture was extracted with ethyl acetate. The obtained crystals were recrystallized from a mixed solvent of ethyl acetate and diethyl ether to give 0.32 g of 6-acetylamino-3-(2-(1-benzyl-4-piperidyl)ethyl)-1,2-benzisoxazole 1/2 hydrate.

$^1$H-NMR($CDCl_3$)$\delta$ : 1.10-2.30(m,9H), 2.23(s,3H), 2.74-3.06(m,4H), 3.51(s,2H), 7.13-7.72(m,8H), 8.00-(s,1H)

6-Acetylamino-3-(2-(1-benzyl-4-piperidyl)ethyl)-1,2-benzisoxazole hydrochloride, melting point 250.6 °C (decomposition) (recrystallized from aqueous ethanol).

$^1$H-NMR($CD_3OD$)$\delta$ : 1.45-1.88(m,5H), 2.05-2.12(m,2H), 2.17(s,3H), 3.02(t,2H), 2.95-3.07(m,2H), 3.46-3.53-(m,2H), 4.28(s,1H), 7.34(d,1H), 7.47-7.55(m,5H), 7.70(d,1H), 8.11(s,1H)

Example 7

6-Amino-3-(2-(1-benzyl-4-piperidyl)ethyl)-1,2-benzisoxazole (0.5 g) and triethylamine (0.18 g) were dissolved in chloroform (5 ml) and phenylacetyl chloride (0.28 g) was added thereto under ice-cooling. After stirring the mixture at room temperature overnight, aqueous sodium bicarbonate was added thereto and the mixture was extracted with ethyl acetate. The extract was washed with water and concentrated under reduced pressure to give crude crystals. The said crystals were recrystallized from a mixed solvent of isopropyl alcohol and toluene to give 0.2 g of 3-(2-(1-benzyl-4-piperidyl)ethyl)-6-phenylacetylamino-1,2-benzisoxazole, melting point 114 - 117 °C.

Reference Example 17

3-(1-Acetyl-4-piperidyl)propionyl chloride (13.9 g) was dissolved in dichloroethane (220 ml) and aluminium chloride (17.0 g) was added thereto under ice-cooling. Stirring of the mixture at said temperature for 1 hour gave a brown solution. The solution was added dropwise to a solution of m-dimethoxybenzene (8.8 g) in dichloroethane (90 ml) at room temperature over 40 minutes. After stirring at room temperature overnight, aluminium chloride (8.5 g) was added thereto. After being stirred at room temperature for 2 hours, the reaction mixture was poured onto ice-water and extracted with chloroform. The extract was washed with water, dried and concentrated under reduced pressure. The obtained oily substance was purified by silica gel chromatography to give 8.8 g of 3-(1-acetyl-4-piperidyl)-2'-hydroxy-4'-methoxypropiophenone.

$^1$H-NMR($CDCl_3$)$\delta$ : 0.92-1.89(m,7H), 2.08(s,3H), 2.70(brs,2H), 2.92(t,2H), 3.83(s,3H), 4.45(brs,2H), 7.59-(d,1H)

Reference Example 18

3-(1-Acetyl-4-piperidyl)-2'-hydroxy-4'-methoxypropiophenone (8.8 g), triethylamine (8.7 g) and hydroxylamine hydrochloride (5.0 g) were treated in a similar manner as in Reference Example 9 to give 6.4 g of 3-(1-acetyl-4-piperidyl)- 2'-hydroxy-4'-methoxypropiophenone oxime, melting point 167 - 168 °C.

Reference Example 19

3-(1-Acetyl-4-piperidyl)-2'-hydroxy-4'-methoxypropiophenone oxime (6.3 g) and acetic anhydride (2.4 ml) were reacted and treated in a similar manner as in Reference Example 10 to give 8.0 g of 3-(1-acetyl-4-piperidyl)- 2'-hydroxy-4'-methoxypropiophenone oxime acetate, melting point 74 - 83 °C.

Reference Example 20

3-(1-Acetyl-4-piperidyl)-2'-hydroxy-4'-methoxypropiophenone oxime acetate (7.5 g) and 60% sodium hydride (0.91 g) were reacted and treated in a similar manner as in Reference Example 11 to give 6 g of 3-(2-(1-acetyl-4-piperidyl)ethyl)-6-methoxy-1,2-benzisoxazole, melting point 89 - 93 °C.

Example 8

3-(2-(1-Acetyl-4-piperidyl)ethyl)-6-methoxy-1,2-benzisoxazole (6 g) was dissolved in 2N hydrochloric acid and refluxed under stirring for 3 hours. The reaction mixture was concentrated under reduced pressure and isopropyl alcohol was added thereto. The precipitated crystals were collected by filtration to give 3.9 g of 3-(2-(4-piperidyl)ethyl)-6-methoxy-1,2-benzisoxazole hydrochloride, melting point 181 - 183 °C.

Example 9

3-(2-(4-Piperidyl)ethyl)-6-methoxy-1,2-benzisoxazole hydrochloride (4.4 g), potassium carbonate (4.5 g) and benzyl chloride (2.1 g) were suspended in dimethylformamide (43 ml) and the suspension was stirred at room temperature for 20 hours. The reaction mixture was concentrated, extracted with ethyl acetate and purified by silica gel chromatography to give 5.3 g of 3-(2-(1-benzyl-4-piperidyl)ethyl)-6-methoxy-1,2-benzisoxazole,melting point 88 - 89 °C.

Example 10

3-(2-(1-Benzyl-4-piperidyl)ethyl)-6-methoxy-1,2-benzisoxazole (4.9 g) was suspended in 47% hydrobromic acid and the mixture was refluxed under stirring for 5 hours. The reaction mixture was concentrated under reduced pressure and aqueous sodium bicarbonate was added thereto. The mixture was extracted with ethyl acetate. The extract was washed with water, dried over magnesium sulfate and concentrated under reduced pressure to give 4.1 g of 3-(2-(1-benzyl-4-piperidyl)ethyl)-6-hydroxy-1,2-benzisoxazole.

$^1$H-NMR($CDCl_3$)$\delta$ : 1.10-2.21(m,9H), 2.70-3.12(m,4H), 3.56(s,2H), 6.64-6.79(m,2H), 7.10-7.38(m,6H), 8.32-(s,1H)

Example 11

Sodium hydride (60%, 0.25 g) was suspended in dimethylformamide and 3-(2-(1-benzyl-4-piperidyl)-ethyl)-6-hydroxy-1,2-benzisoxazole (1.8 g) in dimethylformamide (12 ml) was added thereto at room temperature over 5 minutes. After being stirred at room temperature for 1 hour, the reaction mixture was ice-cooled and N,N-dimethylthiocarbamoyl chloride (1.0 g) was added thereto. After being stirred at room temperature for 1 hour, the reaction mixture was stirred at 50 °C for 2 hours. The reaction mixture was poured into ice-water and extracted with chloroform. The obtained crude crystals were recrystallized from isopropyl ether to give 1.2 g of 3-(2-(1-benzyl-4-piperidyl)ethyl)-6-(N,N-dimethylcarbamoyloxy)-1,2-benzisoxazole, melting point 112 - 113 °C.

Reference Example 21

N-(4-Methoxyphenyl)benzamide (12.5 g), 3-(1-acetyl-4-piperidyl)propionic chloride (12.0 g) and aluminium chloride (25.7 g) were reacted and treated in a similar manner as in Reference Example 8 to give 9.6 g of 3-(1-acetyl-4-piperidyl)-5'-benzoylamino-2'-hydroxypropiophenone, melting point 206 - 209 °C.

Reference Example 22

3-(1-Acetyl-4-piperidyl)-5'-benzoylamino-2'-hydroxypropiophenone (9.6 g), triethylamine (6.2 g) and hydroxylamine hydrochloride (3.4 g) were reacted and treated in a similar manner as in Reference Example 9 to give 7.0 g of 3-(1-acetyl-4-piperidyl)-5'-benzoylamino-2'-hydroxypropiophenone oxime.

$^1$H-NMR($CDCl_3$)$\delta$ : 2.08(s,3H), 3.03(t,2H), 3.50-3.90(m,1H), 4.39-4.69(m,1H), 7.28-7.56(m,4H), 7.78-7.99-(m,3H)

Reference Example 23

3-(1-Acetyl-4-piperidyl)-5'-benzoylamino-2'-hydroxypropiophenone oxime (7.0 g) and acetic anhydride (2.6 g) were reacted and treated in a similar manner as in Reference Example 10 to give 3.8 g of 3-(1-acetyl-4-piperidyl)-5'-benzoylamino-2'-hydroxypropiophenone oxime acetate. This acetate (unpurified) and 60% sodium hydride (0.39 g) were reacted and treated in a similar manner as in Reference Example 11 to give 1.2 g of 3-(2-(1-acetyl-4-piperidyl)ethyl)-5-benzoylamino-1,2-benzisoxazole.

$^1$H-NMR($CDCl_3$)δ : 0.85-1.97(m,7H), 2.80(s,3H), 3.00(t,2H), 3.30-4.72(brs,2H), 7.30-7.58(m,5H), 7.70-7.97-(m,2H), 8.22-8.37(m, 2H)

Example 12

3-(2-(1-Acetyl-4-piperidyl)ethyl)-5-benzoylamino-1,2-benzoyloxazole (1.2 g) and 3N aqueous solution of hydrochloric acid (30 ml) were reacted and treated in a similar manner as in Example 2 to give 0.72 g of 5-amino-3-(2-(4-piperidyl)ethyl)-1,2-benzisoxazole dihydrchloride.

$^1$H-NMR($D_2O$)δ : 1.21-2.20(m,7H), 2.70-3.59(m,4H), 3.14(t,2H), 7.56-7.90(m,3H)

Example 13

5-Amino-3-(2-(4-piperidyl)ethyl)-1,2-benzisoxazole dihydrochloride (0.72 g), potassium carbonate (1.9 g) and benzyl bromide (0.46 g) were reacted and treated in a similar manner as in Example 4 to give 0.53 g of 5-amino-3-(2-(1-benzyl-4-piperidyl)ethyl)-1,2-benzisoxazole.

$^1$H-NMR($CDCl_3$)δ : 1.16-2.38(m,7H), 2.76-2.96(m,4H), 3.48(s,2H), 6.69(d,1H), 6.85(dd,1H), 7.25(s,5H)

Example 14

5-Amino-3-(2-(1-benzyl-4-piperidyl)ethyl)-1,2-benzisoxazole (0.26 g) and acetic anhydride (0.1 g) were reacted and treated in a similar manner as in Example 6 to give 0.4 g of 5-acetylamino-3-(2-(1-benzyl-4-piperidyl)ethyl)-1,2-benzisoxazole as an oily substance. This oily substance was dissolved in ethyl acetate and fumaric acid (0.13 g) was added thereto. The resultant crystals were collected by filtration and recrystallized from ethyl acetate to give 0.18 g of 5-acetylamino-3-(2-(1-benzyl-4-piperidyl)ethyl)-1,2-benzisoxazole fumarate monohydrate.

$^1$H-NMR($CD_3OD$)δ : 2.16(s,3H), 2.72-3.14(m,4H), 4.21(s,2H), 6.66(s,2H), 7.39-7.54(m,8H)

Example 15

5-Amino-3-(2-(1-benzyl-4-piperidyl)ethyl)-1,2-benzisoxazole (0.26 g) and benzoic anhydride (0.27 g) were reacted and treated in a similar manner as in Example 5 to give 0.4 g of 5-benzoylamino-3-(2-(1-benzyl-4-piperidyl)ethyl)-1,2-benzisoxazole as crystals. The crystals were recrystallized from a mixed solvent of isopropyl ether and ethyl acetate and purified, melting point 112 - 113 °C.

Reference Example 24

2-(1-Acetyl-4-piperidyl)acetyl chloride (13 g), 1,2,3-trimethoxybenzene(7.8 g) and aluminium chloride (17 g) were reacted and treated in a similar manner as in Reference Example 17 to give 4.5 g of 2-(1-acetyl-4-piperidyl)-2'-hydroxy-3',4'-dimethoxyacetophenone.

$^1$H-NMR($CDCl_3$)δ : 0.95-1.24(m,2H), 1.63-1.96(m,2H), 2.08(s,3H), 2.10-2.32(m,1H), 2.37-2.75(m,1H), 2.85-(d,2H), 2.90-3.26(m,1H), 3.60-3.75(m,1H), 3.88(s,3H), 3.96(s,3H), 4.41-4.77(m,1H), 6.49(d,1H), 7.45(d,1H), 13.05(s,1H)

Reference Example 25

2-(1-Acetyl-4-piperidyl)-2'-hydroxy-3',4'-dimethoxyacetophenone (4.4 g) and hydroxylamine hydrochloride (2.9 g) were reacted and treated in a similar manner as in Reference Example 9 to give 3.3 g of 2-(1-acetyl-4-piperidyl)-2'-hydroxy-3',4'-dimethoxypropiophenone oxime, melting point 202 - 204 °C.

Reference Example 26

2-(1-Acetyl-4-piperidyl)-2'-hydroxy-3',4'-dimethoxypropiophenone oxime (3.1 g) and acetic anhydride (1.4 g) were reacted and treated in a similar manner as in Reference Example 10 to give 3.8 g of 2-(1-acetyl-4-piperidyl)-2'-hydroxy-3',4'-dimethoxypropiophenone oxime acetate.

$^1$H-NMR($CDCl_3$)δ : 1.14-1.52(m,2H), 1.60-1.88(m,2H), 2.09(s,3H), 1.99-2.21(m,1H), 2.23(s,3H), 2.26-2.63-(m,1H), 2.84(d,2H), 2.92-3.28(m,1H), 3.60-3.83(m,1H), 3.90(s,6H), 4.41-4.77(m,1H), 6.53(d,1H), 7.16(d,1H), 11.47(s,1H)

Reference Example 27

2-(1-Acetyl-4-piperidyl)-2'-hydroxy-3',4'-dimethoxypropiophenone oxime acetate (3.7 g) and 60% sodium hydride (0.4 g) were reacted and treated in a similar manner as in Reference Example 11 to give 2.7 g of 3-(1-acetyl-4-piperidyl)methyl-6,7-dimethoxy-1,2-benzisoxazole, melting point 120 - 121 ° C.

Example 16

3-(1-Acetyl-4-piperidyl)methyl-6,7-dimethoxy-1,2-benzisoxazole (2.6 g) and 2N hydrochloric acid (100 ml) were reacted and treated in a similar manner as in Example 8 to give 1.9 g of 3-(4-piperidyl)methyl-6,7-dimethoxy-1,2-benzisoxazole hydrochloride, melting point 208 - 210 ° C.

Example 17

3-(4-Piperidyl)methyl-6,7-dimethoxy-1,2-benzisoxazole hydrochloride (0.63 g), potassium carbonate (0.6 g) and benzyl chloride (0.30 g) were reacted and treated in a similar manner as in Example 9 to give 6.7 g of 3-(1-benzyl-4-piperidyl)methyl-6,7-dimethoxy-1,2-benzisoxazole as an oily substance. Said oily substance (0.53 g) was dissolved in isopropyl alcohol and fumaric acid (0.48 g) was added thereto. The precipitated crystals were collected by filtration and recrystallized from isopropyl alcohol to give 0.6 g of 3-(1-benzyl-4-piperidyl)methyl-6,7-dimethoxy-1,2-benzisoxazole fumarate, melting point 153 - 154 ° C.

Reference Example 28

3-(1-Acetyl-4-piperidyl)propionyl chloride (4.3 g), 1,2,3-trimethoxybenzene (3.7 g) and aluminium chloride (5.3 g) were reacted and treated in a similar manner as in Reference Example 17 to give 2.8 g of 3-(1-acetyl-4-piperidyl)-2'-hydroxy-3',4'-dimethoxypropiophenone.

$^1$H-NMR($CDCl_3$)$\delta$ : 0.9-2.0(m,7H), 2.15(s,3H), 2.2-3.3(m,4H), 3.6-3.9(m,1H), 3.92(s,3H), 3.97(s,3H), 4.33-4.85(m,3H), 6.5(d,1H,J = 8Hz), 7.5(d,1H,J = 8Hz), 12.5(s,1H)

Reference Example 29

3-(1-Acetyl-4-piperidyl)-2'-hydroxy-3',4'-dimethoxypropiophenone (2.7 g) and hydroxylamine hydrochloride (1.7 g) were reacted and treated in a similar manner as in Reference Example 9 to give 2.7 g of 3-(1-acetyl-4-piperidyl)-2'-hydroxy-3',4'-dimethoxypropiophenone oxime.

$^1$H-NMR($CDCl_3$)$\delta$ : 1.3-2.0(m,7H), 2.15(s,3H), 2.5-3.4(m,4H), 3.6-3.9(m,1H), 3.9(s,6H), 4.3-5.0(m,1H), 6.45(d,1H,J = 8Hz), 7.1(d,1H,J = 8Hz), 9.5(brs,1H)

Reference Example 30

3-(1-Acetyl-4-piperidyl)-2'-hydroxy-3',4'-dimethoxypropiophenone oxime (2.7 g) and acetic anhydride (0.8 g) were reacted and treated in a similar manner as in Reference Example 10 to give 3.4 g of 3-(1-acetyl-4-piperidyl)-2'-hydroxy-3',4'-dimethoxypropiophenone oxime acetate.

$^1$H-NMR($CDCl_3$)$\delta$ : 1.0-1.9(m,7H), 2.1(s,3H), 2.2(s,3H), 2.5-3.3(m,4H), 3.7-3.9(m,4H), 3.9(s,6H), 4.33-4.85-(m,1H), 6.5(d,1H,J = 8Hz), 7.15(d,1H,J = 8Hz), 11.4(s,1H)

Reference Example 31

3-(1-Acetyl-4-piperidyl)-2'-hydroxy-3',4'-dimethoxypropiophenone oxime acetate (3.0 g) and 60% sodium hydride (0.3 g) were reacted and treated in a similar manner as in Reference Example 11 to give 2.1 g of 3-(2-(1-acetyl-4-piperidyl)ethyl)-6,7-dimethoxy-1,2-benzisoxazole.

$^1$H-NMR($CDCl_3$)$\delta$ : 1.0-1.4(m,2H), 1.6-2.0(m,7H), 2.08(s,3H), 2.8-3.1(m,4H), 3.96(s,3H), 4.12(s,3H), 6.96-(d,1H,J = 8Hz), 7.20(d,1H,J = 8Hz)

Example 18

3-(2-(1-Acetyl-4-piperidyl)ethyl)-6,7-dimethoxy-1,2-benzisoxazole(1.6 g) and 2N hydrochloric acid (25 ml) were reacted and treated in a similar manner as in Example 8 to give 1.8 g of 3-(2-(4-piperidyl)ethyl)-6,7-dimethoxy-1,2-benzisoxazole hydrochloride, melting point 175 - 181 ° C (decomposition).

Example 19

3-(2-(4-Piperidyl)ethyl)-6,7-dimethoxy-1,2-benzisoxazole hydrochloride (0.97 g), potassium carbonate (1.0 g) and benzyl chloride (0.45 ml) were reacted and treated in a similar manner as in Example 9 to give 3-(2-(1-benzyl-4-piperidyl)ethyl)-6,7-dimethoxy-1,2-benzisoxazole as crystals. The crystals were recrystallized from a mixed solvent of diisopropyl ether and hexane and purified, melting point 99 - 100 °C. The corresponding hydrochloride, melting point 175 - 179 °C.

Reference Example 32

4-(1-Acetyl-4-piperidyl)butyric chloride (16.0 g), 1,2,3-trimethoxybenzene (11.6 g) and aluminium chloride (27.6 g) were reacted and treated in a similar manner as in Reference Example 17 to give 3.24 g of 4-(1-acetyl-4-piperidyl)-2'-hydroxy-3',4'-dimethoxybutyrophenone.

$^1$H-NMR($CDCl_3$)$\delta$ : 0.9-1.5(m,7H), 1.5-1.9(m,4H), 2.08(s,3H), 2.7-3.0(m,4H), 3.88(s,3H), 3.92(s,3H), 6.45-(d,1H,J=8Hz), 7.45(d,1H,J=8Hz), 12.56(s,1H)

Reference Example 33

4-(1-Acetyl-4-piperidyl)-2'-hydroxy-3',4'-dimethoxypropiophenone (1.1 g) and hydroxylamine hydrochloride (0.6 g) were reacted and treated in a similar manner as in Reference Example 9 to give 1.1 g of 4-(1-acetyl-4-piperidyl)-2'-hydroxy-3',4'-dimethoxypropiophenone oxime.

$^1$H-NMR($CDCl_3$)$\delta$ : 0.9-1.85(m,11H), 2.10(s,3H), 2.7-2.9(m,4H), 3.98(s,6H), 6.45(d,1H,J=8Hz), 7.06-(d,1H,J=8Hz)

Reference Example 34

4-(1-Acetyl-4-piperidyl)-2'-hydroxy-3',4'-dimethoxypropiophenoxime (1.1 g) and acetic anhydride (0.3 g) were reacted and treated in a similar manner as in Reference Example 10 to give 1.4 g of 4-(1-acetyl-4-piperidyl)-2'-hydroxy-3',4'-dimethoxypropiophenone oxime acetate.

$^1$H-NMR($CDCl_3$)$\delta$ : 0.9-1.9(m,11H), 2.08(s,3H), 2.24(s,3H), 2.7-2.95(m,4H), 3.9(s,6H), 6.48(d,1H,J=8Hz), 7.1(d,1H,J=8Hz)

Reference Example 35

4-(1-Acetyl-4-piperidyl)-2'-hydroxy-3',4'-dimethoxypropiophenoxime acetate (1.4 g) and 60% sodium hydride (0.1 g) were reacted and treated in a similar manner as in Reference Example 11 to give 1.1 g of 3-(3-(1-acetyl-4-piperidyl)propyl)-6,7-dimethoxy-1,2-benzisoxazole.

$^1$H-NMR($CDCl_3$)$\delta$ : 0.9-2.9(m,11H), 2.08(s,3H), 2.8-3.0(m,4H), 3.96(s,3H), 4.20(s,3H), 6.92(d,1H,J=8Hz), 7.16(d,1H,J=8Hz)

Example 20

3-(3-(1-Acetyl-4-piperidyl)propyl)-6,7-dimethoxy-1,2-benzisoxazole (0.99 g) and 2N hydrochloric acid (14 ml) were reacted and treated in a similar manner as in Example 8 to give 0.73 g of 3-(3-(4-piperidyl)propyl)-6,7-dimethoxy-1,2-benzisoxazole hydrochloride.

$^1$H-NMR($CDCl_3$)$\delta$ : 1.1-1.6(m,4H), 1.6(s,5H), 2.85(t,2H,J=7Hz), 3.04(brd,2H,J=12Hz), 3.44-(brd,2H,J=12Hz), 3.96(s,3H), 4.08(s,3H), 7.0(d,1H,J=8Hz), 7.3(d,1H,J=8Hz)

Example 21

3-(3-(4-Piperidyl)propyl)-6,7-dimethoxy-1,2-benzisoxazole hydrochloride (0.70 g), potassium carbonate (1.0 g) and benzyl chloride (0.40 ml) were reacted and treated in a similar manner as in Example 9 to give 0.78 g of 3-(3-(1-benzyl-4-piperidyl)propyl)-6,7-dimethoxy-1,2-benzisoxazole as an oily substance. Said oily substance (0.51 g) was dissolved in ethanol and fumaric acid (0.15 g) was added thereto and dissolved. Diethyl ether was added thereto to allow precipitation of crystals. The crystals were recrystallized from a mixed solvent of ethanol and diethyl ether to give 0.49 g of 3-(3-(1-benzyl-4-piperidyl)propyl)-6,7-dimethoxy-1,2-benzisoxazole fumarate, melting point 97 - 100 °C.

Example 22

3-(2-(4-Piperidyl)ethyl)-6,7-dimethoxy-1,2-benzisoxazole hydrochloride (0.33 g), 3-hydroxybenzyl chloride (0.17 g) and triethylamine (0.30 g) were dissolved in 1,2-dichloroethane (5.0 ml) and the mixture was stirred under warming at 40 °C for 5 hours. The reaction mixture was allowed to cool and extracted with ethyl acetate. The extract was washed with water and dried over magnesium sulfate. The solvent was distilled away under reduced pressure. The residue was purified by silica gel chromatography to give 0.30 g of 3-(2-(1-(3-hydroxybenzyl)-4-piperidyl)ethyl)-6,7-dimethoxy-1,2-benzisoxazole as crystals. The crystals were recrystallized from ethyl acetate, melting point 161 °C.

Example 23

3-(2-(1-(3-Hydroxybenzyl)-4-piperidyl)ethyl)-6,7-dimethoxy-1,2-benzisoxazole (0.47 g), methyl isocyanate (0.10 g) and a catalytic amount of triethylamine were dissolved in dimethylformamide (10 ml) and the mixture was stirred at room temperature for 30 minutes. The solvent was distilled away under reduced pressure and the residue was extracted with ethyl acetate. The extract was washed with water and dried over magnesium sulfate. The solvent was distilled away under reduced pressure. The residue was purified by silica gel chromatography to give 0.37 g of 3-(2-(1-(3-(N-methylcarbamoyloxy)benzyl)-4-piperidyl)ethyl)-6,7-dimethoxy-1,2-benzisoxazole. Monohydrochloride was obtained with hydrogen chloride in ether.

$^1$H-NMR($CDCl_3$)$\delta$ : 1.29-2.18(m,6H), 2.81(s,3H), 2.86-3.15(m,3H), 3.40-3.68(m,2H), 3.98(s,3H), 4.07(s,3H), 4.28(s,2H), 7.13-7.62(m,6H)

The compounds obtained by similar procedures are exemplified in the following.

(24) 3-(2-(1-(2-Chlorobenzyl)-4-piperidyl)ethyl)-6,7-dimethoxy-1,2-benzisoxazole, melting point 91 °C

(25) 3-(2-(1-(4-Chlorobenzyl)-4-piperidyl)ethyl)-6,7-dimethoxy-1,2-benzisoxazole, melting point 94 °C

(26) 3-(2-(1-(2-Methoxybenzyl)-4-piperidyl)ethyl)-6,7-dimethoxy-1,2-benzisoxazole fumarate, melting point 163 °C

(27) 3-(2-(1-(4-Methoxybenzyl)-4-piperidyl)ethyl)-6,7-dimethoxy-1,2-benzisoxazole, melting point 107 °C

(28) 3-(2-(1-Benzyl-4-piperidyl)ethyl)-5-methoxy-1,2-benzisoxazole maleate, melting point 139 - 141 °C

(29) 3-(2-(1-Benzyl-4-piperidyl)ethyl)-6-isopropoxyl-1,2-benzisoxazole fumarate, melting point 136 - 137 °C

(30) 3-(2-(1-Benzyl-4-piperidyl)ethyl)-6-propionylamino-1,2-benzisoxazole, melting point 136 - 137 °C; corresponding hydrochloride, melting point 250 - 254 °C (decomposition)

(31) 3-(2-(1-Benzyl-4-piperidyl)ethyl)-6-isobutyrylamino-1,2-benzisoxazole, melting point 110 - 111 °C

(32) 3-(2-(1-Cyclohexylmethyl-4-piperidyl)ethyl)-6,7-dimethoxy-1,2-benzisoxazole, melting point 93 °C

(33) 3-(2-(1-(3-(N-Methylcarbamoyloxy)benzyl)-4-piperidyl)ethyl)-6,7-dimethoxy-1,2-benzisoxazole hydrochloride 2/3 hydrate, melting point 154 °C

(34) 3-(2-(1-(3-Acetylaminobenzyl)-4-piperidyl)ethyl)-6,7-dimethoxy-1,2-benzisoxazole, melting point 98 - 100 °C

(35) 3-(2-(1-Benzyl-4-piperidyl)ethyl)-6-(N-phenylcarbamoyl)oxy-1,2-benzisoxazole, melting point 88 - 91 °C

(36) 6-Acetylamino-3-(2-(1-(3-(N-methylcarbamoyloxy)benzyl-4-piperidyl)ethyl)-1,2-benzisoxazole, melting point 173 - 176 °C

(37) 3-(2-(1-Benzyl-4-piperidyl)ethyl)-4,6-dimethoxy-1,2-benzisoxazole, melting point 136 - 140 °C

(38) 3-(2-(1-Benzyl-4-piperidyl)ethyl)-6-benzyloxy-1,2-benzisoxazole, melting point 100 - 102 °C

(39) 6,7-Dimethoxy-3-(2-(1-(3-nitrobenzyl)-4-piperidyl)ethyl)-1,2-benzisoxazole, melting point 178 - 179 °C

(40) 3-(2-(1-(3-Chlorobenzyl)-4-piperidyl)ethyl)-6,7-dimethoxy-1,2-benzisoxazole, melting point 154 - 156 °C

(41) 3-(2-(1-Benzyl-4-piperidyl)ethyl)-6-(N-methylacetamino)-1,2-benzisoxazole, melting point 116 - 117 °C; corresponding hydrochloride, melting point 201 - 205 °C

(42) 3-(2-(1-Benzyl-4-piperidyl)ethyl)-6-(N-methyl-N-propionylamino)-1,2-benzisoxazole

400MHz NMR($CDCl_3$) $\delta$ : 1.08(3H,t,J=7Hz), 1.35-1.45(4H,m), 1.70-1.90(5H,m), 2.00(2H,m), 2.10-2.20(2H,m), 2.93(2H,m), 3.00(2H,q,J=7Hz), 3.32(3H,s), 3.53(2H,s), 7.13(1H,dd,J=8.2Hz), 7.32(5H,m), 7.39(1H,d,J=2Hz), 7.65(1H,d,J=8Hz)

(43) 7-Benzyloxy-3-(2-(1-benzyl-4-piperidyl)ethyl)-6-methoxy-1,2-benzisoxazole, melting point 154 - 156 °C

(44) 3-(2-(1-Benzyl-4-piperidyl)ethyl)-7-isopropyloxy-6-methoxy-1,2-benzisoxazole fumarate, melting point 108 - 111 °C

(45) 3-(2-(1-Benzyl-4-piperidyl)ethyl)-6-methylamino-1,2-benzisoxazole fumarate, melting point 136 - 144 °C
(46) 6-(N,N-Dimethylamino)-3-(2-(1-benzyl-4-piperidyl)ethyl)-1,2-benzisoxazole fumarate, melting point 178 - 183 °C
(47) 6,7-Dimethoxy-3-(2-(1-(3-(N-methylacetamino)benzyl)-4-piperidyl)ethyl)-1,2-benzisoxazole
$^1$H-NMR($CD_3OD$)$\delta$ : 3.94(s,3H), 4.05(s,3H), 4.27(s,2H), 6.64(s,2H), 6.96-7.62(m,6H)
(48) 3-(2-(1-Benzyl-4-piperidyl)-1-methylethyl)-6,7-dimethoxy-1,2-benzisoxazole, melting point 84 - 85 °C
(49) 3-(2-(1-(2-Acetylaminobenzyl)-4-piperidyl)ethyl)-6,7-dimethoxy-1,2-benzisoxazole
(50) 3-(2-(1-(4-Acetylaminobenzyl)-4-piperidyl)ethyl)-6,7-dimethoxy-1,2-benzisoxazole
(51) 3-(2-(1-(2-(N,N-Dimethylcarbamoyl)benzyl)-4-piperidyl)ethyl)-6,7-dimethoxy-1,2-benzisoxazole
(52) 3-(2-(1-(3-(N,N-Dimethylcarbamoyl)benzyl)-4-piperidyl)ethyl)-6,7-dimethoxy-1,2-benzisoxazole
(53) 3-(2-(1-(4-(N,N-Dimethylcarbamoyl)benzyl)-4-piperidyl)ethyl)-6,7-dimethoxy-1,2-benzisoxazole
(54) 3-(2-(1-(2-(N,N-Diethylcarbamoyl)benzyl)-4-piperidyl)ethyl)-6,7-dimethoxy-1,2-benzisoxazole
(55) 3-(2-(1-(3-(N,N-Diethylcarbamoyl)benzyl)-4-piperidyl)ethyl)-6,7-dimethoxy-1,2-benzisoxazole
(56) 3-(2-(1-(4-(N,N-Diethylcarbamoyl)benzyl)-4-piperidyl)ethyl)-6,7-dimethoxy-1,2-benzisoxazole
(57) 3-(2-(1-(4-(N-Heptylcarbamoyloxy)benzyl)-4-piperidyl)ethyl)-6,7-dimethoxy-1,2-benzisoxazole
(58) 3-(2-(1-(2-(N-Heptylcarbamoyloxy)benzyl)-4-piperidyl)ethyl)-6,7-dimethoxy-1,2-benzisoxazole
(59) 3-(2-(1-Benzyl-4-piperidyl)ethyl)-7-methoxy-1,2-benzisoxazole
(60) 3-(2-(1-Benzyl-4-piperidyl)ethyl)-6-(N-methylcarbamoyl)oxy-1,2-benzisoxazole
(61) 3-(2-(1-Benzyl-4-piperidyl)ethyl)-6-(N-heptylcarbamoyl)oxy-1,2-benzisoxazole
(62) 3-(2-(1-Benzyl-4-piperidyl)ethyl)-6-(N-cyclohexylcarbamoyl)oxy-1,2-benzisoxazole
(63) 3-(2-(1-Benzyl-4-piperidyl)ethyl)-6-(N,N-diethylcarbamoyl)-1,2-benzisoxazole
(64) 3-(2-(1-Benzyl-4-piperidyl)ethyl)-6-(N,N-dimethylcarbamoyl)-1,2-benzisoxazole
(65) 3-(2-(1-Benzyl-4-piperidyl)ethyl)-6-(N-phenylcarbamoyl)-1,2-benzisoxazole
(66) 3-(2-(1-Cyclopentylmethyl-4-piperidyl)ethyl)-6,7-dimethoxy-1,2-benzisoxazole
(67) (+)-3-(2-(1-(1-Phenylethyl)-4-piperidyl)ethyl)-6,7-dimethoxy-1,2-benzisoxazole
(68) (-)-3-(2-(1-(1-Phenylethyl)-4-piperidyl)ethyl)-6,7-dimethoxy-1,2-benzisoxazole
(69) (+)-6-Acetylamino-3-(2-(1-(1-phenylethyl)-4-piperidyl)ethyl)-1,2-benzisoxazole
(70) (-)-6-Acetylamino-3-(2-(1-(1-phenylethyl)-4-piperidyl)ethyl)-1,2-benzisoxazole
(71) 3-(3-(1-Benzyl-4-piperidyl)propyl)-6-isopropoxy-1,2-benzisoxazole
(72) 3-(2-(1-(4-Fluorobenzyl)-4-piperidyl)ethyl)-6-isopropoxy-1,2-benzisoxazole
(73) 3-(2-(1-(4-Chlorobenzyl)-4-piperidyl)ethyl)-6-isopropoxy-1,2-benzisoxazole
(74) 3-(2-(1-Cyclohexylmethyl-4-piperidyl)ethyl)-6-isopropoxy-1,2-benzisoxazole
(75) 3-(2-(1-(2-Naphthylmethyl)-4-piperidyl)ethyl)-6-isopropoxy-1,2-benzisoxazole
(76) 3-(2-(1-(1-Naphthylmethyl)-4-piperidyl)ethyl)-6-isopropoxy-1,2-benzisoxazole
(77) 6-Benzyloxy-3-(2-(1-(4-fluorobenzyl)-4-piperidyl)ethyl)-1,2-benzisoxazole
(78) 6-Benzyloxy-3-(2-(1-(4-chlorobenzyl)-4-piperidyl)ethyl)-1,2-benzisoxazole
(79) 6-Benzyloxy-3-(3-(1-(4-fluorobenzyl)-4-piperidyl)propyl)-1,2-benzisoxazole
(80) 6-Benzyloxy-3-(3-(1-(4-chlorobenzyl)-4-piperidyl)propyl)-1,2-benzisoxazole
(81) 6-Benzyloxy-3-(2-(1-cyclohexylmethyl-4-piperidyl)ethyl)-1,2-benzisoxazole
(82) 6-Benzyloxy-3-(3-(1-cyclohexylmethyl-4-piperidyl)propyl)-1,2-benzisoxazole
(83) 3-(2-(1-(3,4-Dichlorobenzyl)-4-piperidyl)ethyl)-6,7-dimethoxy-1,2-benzisoxazole
(84) 3-(2-(1-Cyclohexylmethyl-4-piperidyl)ethyl)-6,7-dimethoxy-1,2-benzisoxazole
(85) 3-(2-(1-(2-Pyridylmethyl)-4-piperidyl)ethyl)-6,7-dimethoxy-1,2-benzisoxazole
(86) 3-(2-(1-(4-Pyridylmethyl)-4-piperidyl)ethyl)-6,7-dimethoxy-1,2-benzisoxazole
(87) 3-(3-(1-Benzyl-4-piperidyl)propyl)-6-fluoro-1,2-benzisoxazole
(88) 6-Fluoro-3-(3-(1-(2-phenylethyl)-4-piperidyl)propyl)-1,2-benzisoxazole
(89) 3-(3-(1-(2-(4-Fluorophenyl)ethyl)-4-piperidyl)propyl)-6-fluoro-1,2-benzisoxazole
(90) 6-Fluoro-3-(2-(1-(2-phenylethyl)-4-piperidyl)ethyl)-1,2-benzisoxazole
(91) 3-(2-(1-(2-(4-Fluorophenyl)ethyl)-4-piperidyl)ethyl)-6-fluoro-1,2-benzisoxazole
(92) 3-(2-(1-(4-Fluorobenzyl)-4-piperidyl)ethyl)-6-fluoro-1,2-benzisoxazole
(93) 3-(2-(1-(4-Chlorobenzyl)-4-piperidyl)ethyl)-6-fluoro-1,2-benzisoxazole
(94) 3-(3-(1-(4-Fluorobenzyl)-4-piperidyl)propyl)-6-fluoro-1,2 benzisoxazole
(95) 3-(3-(1-(4-Chlorobenzyl)-4-piperidyl)propyl)-6-fluoro-1,2-benzisoxazole
(96) 3-(2-(1-Cyclohexylmethyl-4-piperidyl)ethyl)-6-fluoro-1,2-benzisoxazole
(97) 3-(3-(1-Cyclohexylmethyl-4-piperidyl)propyl)-6-fluoro-1,2-benzisoxazole
(98) 6-Fluoro-3-(2-(1-(2-pyridylmethyl)-4-piperidyl)ethyl)-1,2-benzisoxazole
(99) 6-Fluoro-3-(2-(1-(4-pyridylmethyl)-4-piperidyl)ethyl)-1,2-benzisoxazole

(100) 6-Fluoro-3-(3-(1-(2-pyridylmethyl)-4-piperidyl)propyl)-1,2-benzisoxazole
(101) 6-Fluoro-3-(3-(1-(4-pyridylmethyl)-4-piperidyl)propyl)-1,2-benzisoxazole
(102) 6-Fluoro-3-(3-(1-(2-naphthylmethyl)-4-piperidyl)propyl)-1,2-benzisoxazole
(103) 6-Fluoro-3-(3-(1-(1-naphthylmethyl)-4-piperidyl)propyl)-1,2-benzisoxazole
(104) 6-Fluoro-3-(2-(1-(2-naphthylmethyl)-4-piperidyl)ethyl)-1,2-benzisoxazole
(105) 6-Fluoro-3-(2-(1-(1-naphthylmethyl)-4-piperidyl)ethyl)-1,2-benzisoxazole
(106) 5-Chloro-3-(2-(1-cyclohexylmethyl-4-piperidyl)ethyl)-1,2-benzisoxazole
(107) 3-(2-(1-Benzyl-4-piperidyl)ethyl)-5-methyl-1,2-benzisoxazole
(108) 3-(2-(1-Cyclohexylmethyl-4-piperidyl)ethyl)-5-methyl-1,2-benzisoxazole
(109) 3-(2-(1-Cyclohexylmethyl-4-piperidyl)ethyl)-6-dimethylamino-1,2-benzisoxazole
(110) 3-(2-(1-(1-Phenylethyl)-4-piperidyl)ethyl)-6,7-dimethoxy-1,2-benzisoxazole, melting point 124 - 125 °C
(111) 3-(2-(1-(2-Phenylethyl)-4-piperidyl)ethyl)-6,7-dimethoxy-1,2-benzisoxazole 1/2 hydrate, melting point 85 - 87 °C
(112) 3-(2-(1-(2-Naphthylmethyl)-4-piperidyl)ethyl)-6,7-dimethoxy-1,2-benzisoxazole fumarate, melting point 123 - 124 °C
(113) 3-(2-(1-(1-Naphthylmethyl)-4-piperidyl)ethyl)-6,7-dimethoxy-1,2-benzisoxazole fumarate, melting point 180 - 182 °C
(114) 3-(2-(1-(1-Pyridylmethyl)-4-piperidyl)ethyl)-6,7-dimethoxy-1,2-benzisoxazole fumarate, melting point 127 - 128 °C
(115) 3-(2-(1-Cyclohexylmethyl-4-piperidyl)ethyl)-6-benzyloxy-1,2-benzisoxazole, melting point 119 - 120 °C
(116) 3-(2-(1-Cyclohexylmethyl-4-piperidyl)ethyl)-6-isopropyloxy-1,2-benzisoxazole fumarate, melting point 172 - 173 °C
(117) 3-(2-(1-(1-Naphthylmethyl)-4-piperidyl)ethyl)-6-isopropyloxy-1,2-benzisoxazole
(118) 3-(2-(1-(2-Naphthylmethyl)-4-piperidyl)ethyl)-6-isopropyloxy-1,2-benzisoxazole
(119) 3-(2-(1-Cyclohexylmethyl-4-piperidyl)ethyl)-6-dimethylamino-1,2-benzisoxazole, melting point 107 - 108 °C
(120) 5-Methyl-3-(2-(1-(2-phenylethyl)-4-piperidyl)ethyl)-1,2-benzisoxazole fumarate, melting point 175 - 177 °C
(121) 6-Amino-3-(2-(1-benzyl-4-piperidyl)ethyl)-5-chloro-1,2-benzisoxazole
(122) 6-Acetylamino-3-(2-(1-benzyl-4-piperidyl)ethyl)-5-chloro-1,2-benzisoxazole
(123) 6-Amino-5-chloro-3-(2-(1-(2-phenylethyl)-4-piperidyl)ethyl)-1,2-benzisoxazole
(124) 6-Acetylamino-5-chloro-3-(2-(1-(2-phenylethyl)-4-piperidyl)ethyl)-1,2-benzisoxazole
(125) 3-(2-(1-Allyl-4-piperidyl)ethyl)-6,7-dimethoxy-1,2-benzisoxazole fumarate, melting point 104 - 106 °C
(126) 3-(2-(1-(3-Methyl-2-butenyl)-4-piperidyl)ethyl)-6,7-dimethoxy-1,2-benzisoxazole

Example 127

Sodium hydride (60%, 3.0 g) was suspended in N,N-dimethylformamide (50 ml) and a solution of 3-hydroxy-1,2-benzisoxazole (10 g) in N,N-dimethylformamide (50 ml) was added thereto under ice-cooling over 30 minutes. After stirring the mixture at room temperature for 40 minutes, a solution of 1-benzyloxycarbonyl-4-(2-iodoethyl)piperidine (31.8 g) in N,N-dimethylformamide (50 ml) was added thereto under ice-cooling over 40 minutes. After being stirred at room temperature overnight, the reaction mixture was concentrated. The concentrate was extracted with ethyl acetate. The extract was washed with water, concentrated and purified by silica gel chromatography to give 20 g of 3-(2-(1-benzyloxycarbonyl-4-piperidyl)ethyloxy)-1,2-benzisoxazole.

$^1$H-NMR($CDCl_3$)$\delta$ : 1.08-1.93(m,7H), 2.04-2.35(m,2H), 4.08-4.30(m,2H), 4.50(t,2H), 5.12(s,2H), 7.14-7.65-(m,9H)

Example 128

3-(2-(1-Benzyloxycarbonyl-4-piperidyl)ethyloxy)-1,2-benzisoxazole (19.5 g) was dissolved in acetic acid (40 ml) and 30% hydrogen bromide-acetic acid (50 ml) was added thereto under ice-cooling. After stirring the mixture at room temperature for 1 hour, isopropyl ether was added thereto and precipitated crystals were collected by filtration. Sodium hydrogencarbonate was added to the obtained crystals and the mixture was extracted with ethyl acetate. The extract was dried and concentrated to give 6.2 g of 3-(2-(4-piperidyl)-ethyloxy)-1,2-benzisoxazole.

$^1$H-NMR($CDCl_3$)$\delta$ : 1.15-1.92(m,7H), 2.48-2.75(m,2H), 3.00-3.20(m,2H), 4.28(t,2H), 7.13-7.65(m,4H)

Example 129

3-(2-(4-Piperidyl)ethyloxy)-1,2-benzisoxazole (3.0 g), potassium carbonate (2.4 g) and benzyl chloride (2.2 g) were suspended in acetone (20 ml) and the suspension was stirred at room temperature for 40 hours. The reaction mixture was concentrated and extracted with ethyl acetate. The extract was purified by silica gel chromatography. The obtained oily substance was converted to maleate and recrystallized from a mixed solvent of isopropyl alcohol-ethyl alcohol to give 3.3 g of 3-(2-(1-benzyl-4-piperidyl)ethyloxy)-1,2-benzisoxazole maleate, melting point 160 - 162 ° C.

Example 130

Sodium hydride (60%, 1.2 g) was suspended in N,N-dimethylformamide (20 ml) and a solution of 3-hydroxy-1,2-benzisoxazole (4.1 g) in N,N-dimethylformamide (20 ml) was added under ice-cooling over 20 minutes. After stirring the mixture at room temperature for 50 minutes, a solution of 1-benzyloxycarbonyl-4-iodomethylpiperidine (10.8 g) in N,N-dimethylformamide (20 ml) was added thereto under ice-cooling over 20 minutes. After being stirred at room temperature overnight, the reaction mixture was concentrated. The residue was extracted with ethyl acetate. The extract was washed with water, concentrated and purified by silica gel chromatography to give 4.4 g of 3-(1-benzyloxycarbonyl-4-piperidylmethyloxy)-1,2-benzisoxazole.

$^1$H-NMR($CDCl_3$)$\delta$ : 2.68-3.00(m,2H), 4.09-4.20(m,2H), 4.30(d,2H), 5.13(s,2H), 7.10-7.67(m,9H)

Example 131

3-(1-Benzyloxycarbonyl-4-piperidylmethyloxy)-1,2-benzisoxazole (4.2 g) was dissolved in acetic acid (8.5 ml) and 30% hydrogen bromide-acetic acid (8.5 ml) was added thereto under ice-cooling. After stirring the mixture at room temperature for 3 hours, isopropyl ether was added thereto and the precipitated crystals were washed by decantation. A solution of sodium hydrogencarbonate in water was added to the crystals and the mixture was extracted with chloroform. The extract was dried and concentrated to give 2.2 g of 3-(4-piperidylmethyloxy)-1,2-benzisoxazole.

$^1$H-NMR($CDCl_3$)$\delta$ : 1.14-1.58(m,2H), 2.50-2.84(m,2H), 3.02-3.28(m,2H), 4.30(d,2H), 7.16-7.70(m,4H)

Example 132

3-(4-Piperidylmethyloxy)-1,2-benzisoxazole (2.0 g), potassium carbonate (1.7 g) and benzyl chloride (1.5 g) were suspended in acetone (20 ml) and the suspension was stirred at room temperature for 20 hours. The reaction mixture was concentrated and extracted with ethyl acetate. The extract was purified by silica gel chromatography. The obtained oily substance was converted to maleate and recrystallized from a mixed solvent of isopropyl alcohol-ethanol to give 2.6 g of 3-(1-benzyl-4-piperidylmethyloxy)-1,2-benzisoxazole maleate, melting point 149 - 150 ° C.

Example 133

Sodium hydride (60%, 1.6 g) was suspended in N,N-dimethylformamide (20 ml) and 3-hydroxy-1,2-benzisoxazole (5.0 g) in N,N-dimethylformamide (20 ml) was added thereto under ice-cooling over 25 minutes. After stirring the mixture at room temperature for 1 hour, a solution of 1-benzyloxycarbonyl-4-(3-chloropropyl)piperidine (11.8 g) in N,N-dimethylformamide (20 ml) was added thereto under ice-cooling over 15 minutes. A catalytic amount of sodium iodide was added thereto and the mixture was stirred at 150 ° C overnight and concentrated. The residue was extracted with ethyl acetate and the extract was washed with water and concentrated. The concentrate was purified by silica gel chromatography to give 11 g of 3-(3-(1-benzyloxycarbonyl-4-piperidyl)propyloxy)-1,2-benzisoxazole.

$^1$H-NMR($CDCl_3$)$\delta$ : 2.56-2.92(m,2H), 4.02-4.32(m,2H), 4.42(t,2H), 5.12(s,2H), 7.10-7.68(m,9H)

Example 134

3-(3-(1-Benzyloxycarbonyl-4-piperidyl)propyloxy)-1,2-benzisoxazole (11 g) was dissolved in acetic acid (22 ml) and 30% hydrogen bromide-acetic acid (22 ml) was added thereto under ice-cooling. After stirring the mixture at room temperature for 6 hours, a mixed solvent of isopropyl ether-hexane was added thereto

and the resultant oily substance was washed by decantation. A solution of sodium hydrogencarbonate in water was added in the oily substance and the mixture was extracted with chloroform. The extract was dried and concentrated to give 4.7 g of 3-(3-(4-piperidyl)propyloxy)-1,2-benzisoxazole.

$^1$H-NMR($CDCl_3$)$\delta$ : 1.04-2.10(m,7H), 2.59-3.02(m,2H), 3.25-3.52(m,2H), 4.44(t,3H), 7.16-7.68(m,4H)

Example 135

3-(3-(4-Piperidyl)propyloxy)-1,2-benzisoxazole (2.0 g), potassium carbonate (1.5 g) and benzyl chloride (1.4 g) were suspended in a mixed solvent of acetone (20 ml) and N,N-dimethylformamide (5 ml) and the suspension was stirred at room temperature for 19 hours. The reaction mixture was concentrated, extracted with ethyl acetate and purified by silica gel chromatography. The obtained oily substance was converted to maleate and the maleate was recrystallized from isopropyl alcohol to give 1.3 g of 3-(3-(1-benzyl-4-piperidyl)propyloxy)-1,2-benzisoxazole maleate, melting point 143 - 145 °C.

The compounds obtained by similar procedures are exemplified in the following.

(136) 3-(2-(1-Benzyl-4-piperidyl)ethyloxy)-6-fluoro-1,2-benzisoxazole
(137) 6-Fluoro-3-(2-(1-(2-phenylethyl)-4-piperidyl)ethyloxy)-1,2-benzisoxazole
(138) 6-Fluoro-3-(2-(1-(2-(4-fluorophenyl)ethyl)-4-piperidyl)ethyloxy)-1,2-benzisoxazole
(139) 6-Fluoro-3-(2-(1-(4-fluorobenzyl)-4-piperidyl)ethyloxy)-1,2-benzisoxazole
(140) 3-(2-(1-(4-Chlorobenzyl)-4-piperidyl)ethyloxy)-6-fluoro-1,2-benzisoxazole
(141) 6-Fluoro-3-(3-(1-(4-fluorobenzyl)-4-piperidyl)propyloxy)-1,2-benzisoxazole
(142) 3-(3-(1-(4-Chlorobenzyl)-4-piperidyl)propyloxy)-6-fluoro-1,2-benzisoxazole
(143) 3-(2-(1-Cyclohexylmethyl-4-piperidyl)ethyloxy)-6-fluoro-1,2-benzisoxazole
(144) 3-(3-(1-Cyclohexylmethyl-4-piperidyl)propyloxy)-6-fluoro-1,2-benzisoxazole
(145) 6-Fluoro-3-(2-(1-(2-pyridylmethyl)-4-piperidyl)ethyloxy)-1,2-benzisoxazole
(146) 6-Fluoro-3-(2-(1-(4-pyridylmethyl)-4-piperidyl)ethyloxy)-1,2-benzisoxazole
(147) 6-Fluoro-3-(3-(1-(2-pyridylmethyl)-4-piperidyl)propyloxy)-1,2-benzisoxazole
(148) 6-Fluoro-3-(3-(1-(4-pyridylmethyl)-4-piperidyl)propyloxy)-1,2-benzisoxazole
(149) 6-Fluoro-3-(2-(1-(1-naphthylmethyl)-4-piperidyl)ethyloxy)-1,2-benzisoxazole
(150) 6-Fluoro-3-(2-(1-(2-naphthylmethyl)-4-piperidyl)ethyloxy)-1,2-benzisoxazole
(151) 6-Fluoro-3-(3-(1-(1-naphthylmethyl)-4-piperidyl)propyloxy)-1,2-benzisoxazole
(152) 6-Fluoro-3-(3-(1-(2-naphthylmethyl)-4-piperidyl)propyloxy)-1,2-benzisoxazole
(153) 3-(2-(1-Benzyl-4-piperidyl)ethyloxy)-5-chloro-1,2-benzisoxazole
(154) 5-Chloro-3-(2-(1-cyclohexylmethyl-4-piperidyl)ethyloxy)-1,2-benzisoxazole
(155) 5-Chloro-3-(2-(1-(1-naphthylmethyl)-4-piperidyl)ethyloxy)-1,2-benzisoxazole
(156) 5-Chloro-3-(2-(1-(2-naphthylmethyl)-4-piperidyl)ethyloxy)-1,2-benzisoxazole
(157) 3-(2-(1-Benzyl-4-piperidyl)ethyloxy)-5-methyl-1,2-benzisoxazole
(158) 5-Methyl-3-(2-(1-(2-phenylethyl)-4-piperidyl)ethyloxy)-1,2-benzisoxazole fumarate, melting point 155 - 156 °C
(159) 3-(2-(1-(2-(4-Fluorophenyl)ethyl)-4-piperidyl)ethyloxy)-5-methyl-1,2-benzisoxazole
(160) 3-(2-(1-Cyclohexylmethyl-4-piperidyl)ethyloxy)-5-methyl-1,2-benzisoxazole fumarate, melting point 178 - 179 °C
(161) 3-(2-(1-Benzyl-4-piperidyl)ethyloxy)-6-methyl-1,2-benzisoxazole
(162) 6-Methyl-3-(3-(1-(2-phenylethyl)-4-piperidyl)propyloxy)-1,2-benzisoxazole
(163) 3-(2-(1-(2-(4-Fluorophenyl)ethyl)-4-piperidyl)propyloxy)-6-methyl-1,2-benzisoxazole
(164) 3-(2-(1-Cyclohexylmethyl-4-piperidyl)ethyloxy)-6-methyl-1,2-benzisoxazole fumarate, melting point 182 - 184 °C
(165) 3-(3-(1-Cyclohexylmethyl-4-piperidyl)propyloxy)-6-methyl-1,2-benzisoxazole
(166) 6-Methyl-3-(2-(1-(1-naphthylmethyl)-4-piperidyl)ethyloxy)-1,2-benzisoxazole
(167) 6-Methyl-3-(2-(1-(2-naphthylmethyl)-4-piperidyl)ethyloxy)-1,2-benzisoxazole
(168) 5-Methyl-3-(2-(1-(1-naphthylmethyl)-4-piperidyl)ethyloxy)-1,2-benzisoxazole fumarate, melting point 191 - 193 °C
(169) 5-Methyl-3-(2-(1-(2-naphthylmethyl)-4-piperidyl)ethyloxy)-1,2-benzisoxazole fumarate, melting point 176 - 178 °C
(170) 5-Methyl-3-(2-(1-benzyl-4-piperidyl)ethyloxy)-1,2-benzisoxazole fumarate, melting point 162 - 163 °C
(171) 5-Methyl-3-(2-(1-(2-pyridylmethyl)-4-piperidyl)ethyloxy)-1,2-benzisoxazole fumarate, melting point 145 - 146 °C

(172) 6-Methyl-3-(2-(1-(2-phenylethyl)-4-piperidyl)ethyloxy)-1,2-benzisoxazole fumarate, melting point 158 - 159 ° C
(173) 6-Chloro-3-(2-(1-(2-phenylethyl)-4-piperidyl)ethyloxy)-1,2-benzisoxazole fumarate, melting point 187 - 189 ° C
(174) 6-Chloro-3-(2-(1-(2-phenylethyl)-4-piperidyl)propyloxy)-1,2-benzisoxazole fumarate, melting point 165 - 166 ° C
(175) 3-(2-(1-(2-Phenylethyl)-4-piperidyl)ethyloxy)-1,2-benzisoxazole fumarate, melting point 149 - 150 ° C
(176) 6-Chloro-3-(2-(1-(2-phenylethyl)-4-piperidyl)methoxy)-1,2-benzisoxazole fumarate, melting point 193 - 195 ° C
(177) 6-Chloro-3-(2-(1-cyclohexylmethyl-4-piperidyl)ethyloxy)-1,2-benzisoxazole, melting point 134 - 136 ° C
(178) 6-Chloro-3-(2-(1-cyclohexylmethyl-4-piperidyl)methoxy)-1,2-benzisoxazole fumarate
(179) 5-Chloro-3-(2-(1-(2-phenylethyl)-4-piperidyl)methoxy)-1,2-benzisoxazole fumarate
(180) 5-Chloro-3-(2-(1-cyclohexylmethyl-4-piperidyl)ethoxy)-1,2-benzisoxazole fumarate
(181) 5-Chloro-3-(2-(1-cyclohexylmethyl-4-piperidyl)methoxy)-1,2-benzisoxazole fumarate
(182) 5-Chloro-3-(2-(1-(2-phenylethyl)-4-piperidyl)propyloxy)-1,2-benzisoxazole fumarate
(183) 3-(2-(1-Allyl-4-piperidyl)ethyloxy)-6-chloro-1,2-benzisoxazole, melting point 43 - 45 ° C
(184) 6-Chloro-3-(2-(1-(3-methyl-2-butenyl)-4-piperidyl)ethyloxy)-1,2-benzisoxazole
(185) 3-(2-(1-Cyclohexyl-4-piperidyl)ethyloxy)-1,2-benzisoxazole
(186) 3-(2-(1-Cyclohexyl-4-piperidyl)ethyloxy)-6-methyl-1,2-benzisoxazole

Example 187

Sodium hydride (60%, 3.0 g) was suspended in N,N-dimethylformamide (50 ml) and a solution of 1,2-benzisoxazol-3(2H)-one (10 g) in N,N-dimethylformamide (50 ml) was added thereto under ice-cooling over 30 minutes. After stirring the mixture at room temperature for 40 minutes, a solution of 1-benzyloxycarbonyl-4-(2-iodoethyl)piperidine (31.8 g) in N,N-dimethylformamide (50 ml) was added thereto under ice-cooling over 40 minutes. After being stirred at room temperature overnight, the reaction mixture was concentrated. The residue was extracted with ethyl acetate. The extract was washed with water, concentrated and purified by silica gel column chromatography to give 10.3 g of 2-(2-(1-benzyloxycarbonyl-4-piperidyl)ethyl)-1,2-benzisoxazol-3(2H)-one, melting point 89 - 91 ° C.

Example 188

2-(2-(1-Benzyloxycarbonyl-4-piperidyl)ethyl)-1,2-benzisoxazol-3(2H)-one (10 g) was dissolved in acetic acid (20 ml) and 30% hydrogen bromide-acetic acid (20 ml) was added thereto under ice-cooling. After stirring the mixture at room temperature for 2 hours, isopropyl ether was added thereto to give a sediment of an oily substance. The supernatant was removed by decantation and a solution of sodium hydrogencarbonate in water was added to the residue. The mixture was extracted with chloroform. The extract was dried and concentrated to give 3.0 g of 2-(2-(4-piperidyl)ethyl)-1,2-benzisoxazol-3(2H)-one.

$^1$H-NMR($CDCl_3$)$\delta$ : 1.05-1.98(m,7H), 2.48-2.82(m,2H), 3.04-3.28(m,2H), 4.05(t,2H), 7.11-7.94(m,4H)

Example 189

2-(2-(4-Piperidyl)ethyl)-1,2-benzisoxazol-3(2H)-one (3.0 g), potassium carbonate (2.4 g) and benzyl chloride (2.2 g) were suspended in a mixed solvent of acetone (20 ml) and N,N-dimethylformamide (30 ml) and the suspension was stirred at room temperature for 40 minutes. The reaction mixture was concentrated and extracted with ethyl acetate. The extract was purified by silica gel column chromatography. The obtained oily substance was converted to fumarate and recrystallized from a mixed solvent of isopropyl alcohol and ethyl acetate to give 1.4 g of 2-(2-(1-benzyl-4-piperidyl)ethyl)-1,2-benzisoxazol-3(2H)-one fumarate, melting point 155 - 156 ° C.

Example 190

1,2-Benzisoxazol-3(2H)-one (4.1 g) and 1-benzyloxycarbonyl-4-iodomethylpiperidine (10.8g) were reacted and treated in the same manner as in Example 189 to give 1.9 g of 2-(1-benzyloxycarbonyl-4-piperidylmethyl)-1,2-benzisoxazol-3(2H)-one, melting point 95 - 98 ° C.

Example 191

2-(1-Benzyloxycarbonyl-4-piperidylmethyl)-1,2-benzisoxazol-3(2H)-one (1.8 g) was dissolved in acetic acid (3.6 ml) and 30% hydrogen bromide-acetic acid (3.6 ml) was added thereto under ice-cooling. After stirring the mixture at room temperature for 1 hour, isopropyl ether was added thereto to afford a sediment of an oily substance. The supernatant was removed by decantation and a solution of sodium hydrogencarbonate in water was added to the residue. The mixture was extracted with chloroform. The extract was dried and concentrated to give 1.0 g of 2-(4-piperidylmethyl)-1,2-benzisoxazol-3(2H)-one, melting point 104 - 106 °C.

Example 192

2-(4-Piperidylmethyl)-1,2-benzisoxazol-3(2H)-one (0.9 g) and benzyl chloride (0.7 g) were reacted in the same manner as in Example 191 to give 0.52 g of 2-(1-benzyl-4-piperidylmethyl)-1,2-benzisoxasol-3(2H)-one, melting point 110 - 112 °C.

Example 193

1,2-Benzisoxazol-3(2H)-one (5.0 g) and 1-benzyloxycarbonyl-4-(3-chloropropyl)piperidine (11.8 g) were reacted and treated in the same manner as in Example 187 to give 4.7 g of 2-(3-(1-benzyloxycarbonyl-4-piperidyl)propyl)-1,2-benzisoxazol-3(2H)-one.

$^1$H-NMR($CDCl_3$)$\delta$ : 0.84-2.05(m,9H), 1.54-1.91(m,2H), 4.00-4.28(m,2H), 4.03(t,2H), 5.10(s,2H), 7.14-7.88-(m,9H)

Example 194

2-(3-(1-Benzyloxycarbonyl-4-piperidyl)propyl)-1,2-benzisoxazol-3(2H)-one (4.7 g) and 30% hydrogen bromide-acetic acid (10 ml) were reacted and treated in the same manner as in Example 191 to give 2.8 g of 2-(3-(4-piperidyl)propyl)-1,2-benzisoxazol-3(2H)-one.

$^1$H-NMR($CDCl_3$)$\delta$ : 0.78-2.10(m,9H), 2.55-3.00(m,2H), 3.18-3.52(m,2H), 4.04(t,2H), 7.14-7.88(m,9H)

Example 195

2-(3-(4-Piperidyl)propyl)-1,2-benzisoxazol-3(2H)-one (1.5 g) and benzyl chloride (1.0 g) were reacted in the same manner as in Example 189 to give 0.6 g of 2-(3-(1-benzyl-4-piperidyl)propyl)-1,2-benzisoxazol-3-(2H)-one fumarate, melting point 133 - 134 °C.

Example 196

6-Amino-1,2-benzisoxazol-3(2H)-one (0.8 g) and 1-benzyloxycarbonyl-4-(3-iodopropyl)piperidine (2.1 g) were reacted and treated in the same manner as in Example 187 to give 1.5 g of 6-amino-2-(3-(1-benzyloxycarbonyl-4-piperidyl)propyl)-1,2-benzisoxazol-3(2H)-one, melting point 120 - 123 °C.

Example 197

6-Amino-2-(3-(1-benzyloxycarbonyl-4-piperidyl)propyl)-1,2-benzisoxazol-3(2H)-one (1.5 g) was dissolved in pyridine (30 ml) and benzoyl chloride (0.6 g) was added thereto. After being stirred at room temperature for 8 hours, the reaction mixture was concentrated and purified by silica gel column chromatography to give 1.7 g of 6-benzoylamino-2-(3-(1-benzyloxycarbonyl-4-piperidyl)propyl)-1,2-benzisoxazol-3(2H)-one.

$^1$H-NMR($CDCl_3$)$\delta$ : 0.75-2.05(m,9H), 2.40-3.05(m,2H), 3.80(t,2H), 3.85-4.41(m,2H), 5.09(s,2H), 6.88(d,1H), 7.20-8.00(m,12H), 8.45(s,1H)

Example 198

6-Benzoylamino-2-(3-(1-benzyloxycarbonyl-4-piperidyl)propyl)-1,2-benzisoxazol-3(2H)-one (1.7 g) was dissolved in acetic acid (1.7 g) and 30% hydrogen bromide-acetic acid (8 ml) was added thereto under ice-cooling. After stirring the mixture at room temperature for 2.5 hours, isopropyl ether was added thereto to afford a sediment of an oily substance. The supernatant was removed by decantation and the residue was

dried under reduced pressure. Thereto were added potassium carbonate (1.0 g), benzyl chloride (0.45 g) and then N,N-dimethylformamide (7 ml). The mixture was stirred at room temperature for 12 hours. The reaction mixture was concentrated and extracted with ethyl acetate. The obtained crude crystals were recrystallized from ethyl acetate to give 0.8 g of 6-benzoylamino-2-(3-(1-benzyl-4-piperidyl)propyl)-1,2-benzisoxazol-3(2H)-one, melting point 163 - 165 °C.

The compounds obtained by similar procedures are exemplified in the following.

(199) 2-(2-(1-Benzyl-4-piperidyl)ethyl)-6-fluoro-1,2-benzisoxazol-3(2H)-one
(200) 6-Fluoro-2-(2-(1-(2-phenylethyl)-4-piperidyl)ethyl)-1,2-benzisoxazol-3(2H)-one
(201) 6-Fluoro-2-(2-(1-(2-(4-fluorophenyl)ethyl)-4-piperidyl)ethyl)-1,2-benzisoxazol-3(2H)-one
(202) 6-Fluoro-2-(2-(1-(4-fluorobenzyl)-4-piperidyl)ethyl)-1,2-benzisoxazol-3(2H)-one
(203) 2-(2-(1-(4-Chlorobenzyl)-4-piperidyl)ethyl)-6-fluoro-1,2-benzisoxazol-3(2H)-one
(204)6-Fluoro-2-(3-(1-(4-fluorobenzyl)-4-piperidyl)propyl)-1,2-benzisoxazol-3(2H)-one
(205) 2-(3-(1-(4-Chlorobenzyl)-4-piperidyl)propyl)-6-fluoro-1,2-benzisoxazol-3(2H)-one
(206) 2-(2-(1-Cyclohexylmethyl-4-piperidyl)ethyl)-6-fluoro-1,2-benzisoxazol-3(2H)-one
(207) 2-(3-(1-Cyclohexylmethyl-4-piperidyl)propyl)-6-fluoro-1,2-benzisoxazol-3(2H)-one
(208) 6-Fluoro-2-(2-(1-(2-pyridylmethyl)-4-piperidyl)ethyl)-1,2-benzisoxazol-3(2H)-one
(209) 6-Fluoro-2-(2-(1-(4-pyridylmethyl)-4-piperidyl)ethyl)-1,2-benzisoxazol-3(2H)-one
(210) 6-Fluoro-2-(3-(1-(2-pyridylmethyl)-4-piperidyl)propyl)-1,2-benzisoxazol-3(2H)-one
(211) 6-Fluoro-2-(3-(1-(4-pyridylmethyl)-4-piperidyl)propyl)-1,2-benzisoxazol-3(2H)-one
(212) 6-Fluoro-2-(2-(1-(1-naphthylmethyl)-4-piperidyl)ethyl)-1,2-benzisoxazol-3(2H)-one
(213) 6-Fluoro-2-(2-(1-(2-naphthylmethyl)-4-piperidyl)ethyl)-1,2-benzisoxazol-3(2H)-one
(214) 6-Fluoro-2-(3-(1-(1-naphthylmethyl)-4-piperidyl)propyl)-1,2-benzisoxazol-3(2H)-one
(215) 6-Fluoro-2-(3-(1-(2-naphthylmethyl)-4-piperidyl)propyl)-1,2-benzisoxazol-3(2H)-one
(216) 2-(2-(1-Benzyl-4-piperidyl)ethyl)-5-chloro-1,2-benzisoxazol-3(2H)-one
(217) 5-Chloro-2-(2-(1-cyclohexylmethyl-4-piperidyl)ethyl)-1,2-benzisoxazol-3(2H)-one
(218) 5-Chloro-2-(2-(1-(1-naphthylmethyl)-4-piperidyl)ethyl)-1,2-benzisoxazol-3(2H)-one
(219) 5-Chloro-2-(2-(1-(2-naphthylmethyl)-4-piperidyl)ethyl)-1,2-benzisoxazol-3(2H)-one
(220) 2-(2-(1-Benzyl-4-piperidyl)ethyl)-5-methyl-1,2-benzisoxazol-3(2H)-one
(221) 5-Methyl-2-(2-(1-(2-phenylethyl)-4-piperidyl)ethyl)-1,2-benzisoxazol-3(2H)-one fumarate, melting point 153 - 154 °C
(222) 2-(2-(1-(2-(4-Fluorophenyl)ethyl)-4-piperidyl)ethyl)-5-methyl-1,2-benzisoxazol-3(2H)-one
(223) 2-(2-(1-Cyclohexylmethyl-4-piperidyl)ethyl)-5-methyl-1,2-benzisoxazol-3(2H)-one
(224) 2-(2-(1-Benzyl-4-piperidyl)ethyl)-6-methyl-1,2-benzisoxazol-3(2H)-one
(225) 6-Methyl-2-(3-(1-(2-phenylethyl)-4-piperidyl)propyl)-1,2-benzisoxazol-3(2H)-one
(226) 2-(3-(1-(2-(4-Fluorophenyl)ethyl)-4-piperidyl)propyl)-6-methyl-1,2-benzisoxazol-3(2H)-one
(227) 2-(2-(1-Cyclohexylmethyl-4-piperidyl)ethyl)-6-methyl-1,2-benzisoxazol-3(2H)-one fumarate, melting point 159 - 161 °C
(228) 2-(3-(1-Cyclohexylmethyl-4-piperidyl)propyl)-6-methyl-1,2-benzisoxazol-3(2H)-one
(229) 6-Methyl-2-(2-(1-(1-naphthylmethyl)-4-piperidyl)ethyl)-1,2-benzisoxazol-3(2H)-one
(230) 6-Methyl-2-(2-(1-(2-naphthylmethyl)-4-piperidyl)ethyl)-1,2-benzisoxazol-3(2H)-one
(231) 2-(2-(1-Cyclohexylmethyl-4-piperidyl)ethyl)-5-methyl-1,2-benzisoxazol-3(2H)-one fumarate, melting point 158 - 159 °C
(232) 6-Methyl-2-(2-(1-(2-phenylethyl)-4-piperidyl)ethyl)-1,2-benzisoxazol-3(2H)-one fumarate, melting point 152 - 153 °C
(233) 6-Chloro-2-(2-(1-(2-phenylethyl)-4-piperidyl)ethyl)-1,2-benzisoxazol-3(2H)-one 3/2 fumarate, melting point 136 - 138 °C
(234) 6-Chloro-2-(2-(1-(2-phenylethyl)-4-piperidyl)methyl)-1,2-benzisoxazol-3(2H)-one fumarate, melting point 223 °C (decomposition)
(235) 6-Chloro-2-(2-(1-cyclohexylmethyl-4-piperidyl)ethyl)-1,2-benzisoxazol-3(2H)-one
(236) 6-Chloro-2-(2-(1-cyclohexylmethyl-4-piperidyl)methyl)-1,2-benzisoxazol-3(2H)-one
(237) 5-Chloro-2-(2-(1-(2-phenylethyl)-4-piperidyl)ethyl)-1,2-benzisoxazol-3(2H)-one
(238) 2-(2-(1-Allyl-4-piperidyl)ethyl)-5-methyl-1,2-benzisoxazol-3(2H)-one L-tartrate monohydrate, melting point 154 - 156 °C
(239) 2-(2-(1-(3-Methyl-2-butenyl)-4-piperidyl)ethyl)-5-methyl-1,2-benzisoxazol-3(2H)-one
(240) 6-Benzoylamino-2-(3-(1-benzyl-4-piperidyl)ethyl)-1,2-benzisoxazol-3(2H)-one
(241) 6-Amino-5-chloro-3-(2-(1-benzyl-4-piperidyl)ethyl)-1,2-benzisoxazole fumarate 1/2 hydrate, melting point 154 - 157 °C

(242) 6-Amino-5-chloro-3-(2-(1-(2-phenylethyl)-4-piperidyl)ethyl)-1,2-benzisoxazole fumarate, melting point 195 - 197 °C
(243) 6-Acetylamino-5-chloro-3-(2-(1-benzyl-4-piperidyl)ethyl)-1,2-benzisoxazole fumarate
$^1$H-NMR($CD_3OD$)δ : 2.27(s,3H), 4.26(s,2H), 6.70(s,2H), 7.49(s,5H), 7.93(s,1H), 8.23(s,1H)
(244) 6-Acetylamino-5-chloro-3-(2-(1-(2-phenylethyl)-4-piperidyl)ethyl)-1,2-benzisoxazole fumarate
$^1$H-NMR($CD_3OD$)δ : 2.25(s,3H), 3.44-3.70(m,2H), 6.68(s,2H), 7.27(s,5H), 7.92(s,1H), 8.21(s,1H)
(245) 6-Acetoxy-3-(2-(1-benzyl-4-piperidyl)ethyl)-1,2-benzisoxazole, melting point 93 - 95 °C
(246) 6-Propionyloxy-3-(2-(1-benzyl-4-piperidyl)ethyl)-1,2-benzisoxazole fumarate, melting point 134 - 136 °C
(247) 3-(2-(1-Benzyl-4-piperidyl)ethyl)-6-(N,N-dimethylcarbamoyloxy)-1,2-benzisoxazole, melting point 113 - 115 °C
(248) 3-(2-(1-Benzyl-4-piperidyl)ethyl)-6-(N-methylcarbamoyloxy)-1,2-benzisoxazole
(249) 3-(2-(1-Cyclohexylmethyl-4-piperidyl)ethyloxy)-1,2-benzisoxazole fumarate, melting point 168 - 169 °C
(250) 3-(2-(1-Benzyl-4-piperidyl)ethylthio)-5-methyl-1,2-benzisoxazole
$^1$H-NMR($CDCl_3$)δ : 1.06-2.10(m,9H), 2.46(s,3H), 2.62-2.99(m,2H), 3.30(t,2H), 3.50(s,2H), 7.12-7.51-(m,8H)
(251) 3-(2-(1-Benzyl-4-piperidyl)ethyloxy)-1,2-benzisoxazole fumarate
(252) 6-Benzoylamino-2-(2-(1-benzyl-4-piperidyl)ethyl)-1,2-benzisoxazol-3(2H)-one, melting point 172 - 174 °C
(253) 3-(2-(1-(2-Cyclohexylethenyl)-4-piperidyl)ethyl)-5-methyl-1,2-benzisoxazole
(254) 5-Methyl-3-(2-(1-styryl-4-piperidyl)ethyl)-1,2-benzisoxazole
(255) 5-Methyl-3-(2-(1-(2-(2-naphthyl)ethenyl)-4-piperidyl)ethyl)-1,2-benzisoxazole
(256) 3-(2-(1-(2-Cyclohexylethenyl)-4-piperidyl)ethyloxy)-5-methyl-1,2-benzisoxazole
(257) 5-Methyl-3-(2-(1-styryl-4-piperidyl)ethyloxy)-1,2-benzisoxazole
(258) 5-Methyl-3-(2-(1-(2-(2-naphthyl)ethenyl)-4-piperidyl)ethyloxy)-1,2-benzisoxazole
(259) 2-(2-(1-(2-Cyclohexylethenyl)-4-piperidyl)ethyl)-5-methyl-1,2-benzisoxazol-3(2H)-one
(260) 5-Methyl-2-(2-(1-styryl-4-piperidyl)ethyl)-1,2-benzisoxazol-3(2H)-one
(261) 5-Methyl-2-(2-(1-(2-(2-naphthyl)ethenyl)-4-piperidyl)ethyl)-1,2-benzisoxazol-3(2H)-one

Formulation Example

Tablets containing Compound (I) (10.0 mg) can be prepared according to the following formulation.

| | |
|---|---|
| Compound (I) | 10.0 mg |
| Lactose | 30.0 mg |
| Corn starch | 18.8 mg |
| Crystalline cellulose | 28.0 mg |
| Hydroxypropylcellulose | 1.0 mg |
| Talc | 2.0 mg |
| Magnesium stearate | 0.2 mg |
| | 90.0 mg |

The Compound (I) is pulverized by an atomizer to prepare a fine powder having an average particle size of not more than 10 μ. The Compound (I), lactose, corn starch and crystalline cellulose are mixed in a kneader. Hydroxypropylcellulose is added thereto and the mixture is kneaded for 20 minutes. The kneaded mixture is granulated through a sieve of 200 mesh and dried in a 50 °C hot air dryer to a water content of 3 - 4%. After passing the granules through a 24 mesh sieve, talc and magnesium stearate are mixed together and the mixture is compressed with a 6 mm-diameter flat pounder by a rotary compressor to give tablets.

While the present invention has been adequately and sufficiently described in the foregoing specification and examples, it is susceptible to various modifications and alternative forms falling within the spirit and scope of the present invention.

## Claims

**1.** A benzisoxazole compound of the formula

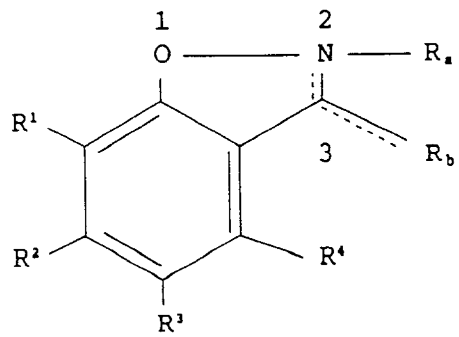

(I)

or a pharmaceutically acceptable salt thereof wherein:
when --------- bond between 2- and 3-positions is a single bond,

$R_a$ is a group of the formula

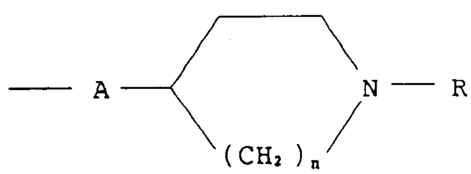

[wherein:

R is hydrogen, alkyl, alkenyl, cycloalkylalkyl, cycloalkylalkenyl, phenylalkyl, phenylalkenyl, naphthylalkyl or naphthylalkenyl,

A is a straight- or branched chain alkylene, and

n is 1, 2 or 3]; and

$R_b$ is oxygen, and

when --------- bond between 2- and 3-positions is a double bond,

$R_a$ is void; and

$R_b$ is a group of the formula

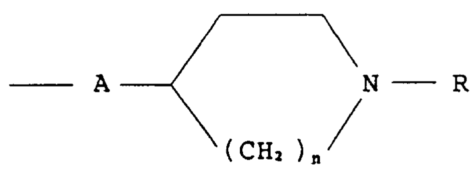

[wherein each symbol is as defined above], or a group of the formula

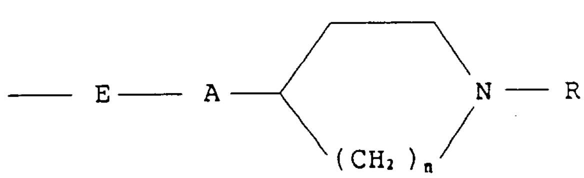

[wherein:

E is oxygen or sulfur and

other symbols are as defined above]; and

$R^1$, $R^2$, $R^3$ and $R^4$ are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)_mR^5$, $-NHSO_2R^5$, $-CONR^6R^7$, $-NR^6R^7$, $-OCONR^6R^7$, $-OCSNR^6R^7$, $-SO_2NR^6R^7$

or $-COOR^8$ or adjacent groups from $R^1$, $R^2$, $R^3$ and $R^4$ may be bonded to form $-O(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted,

[wherein:

$R^5$ is alkyl, phenyl or phenylalkyl,

$R^6$ and $R^7$ are the same or different and each is hydrogen, alkyl, phenyl, phenylalkyl or a group bonded with the adjacent nitrogen atom to form a heterocyclic ring,

$R^8$ is hydrogen, alkyl, phenyl or phenylalkyl,

$R^9$ is hydrogen, alkyl, phenylalkyl or acyl,

m is 0, 1 or 2, and

p, q, r and s are the same or different and each is 1, 2 or 3];

wherein:

in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ and $-COOR^8$ - [wherein $R^5$, $R^6$, $R^7$, $R^8$ and m are as defined above].

2. A benzisoxazole compound of Claim 1 or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl.

3. A benzisoxazole compound of Claim 1 or a pharmaceutically acceptable salt thereof, wherein R is hydrogen.

4. A benzisoxazole compound of Claim 1 or a pharmaceutically acceptable salt thereof, having the formula

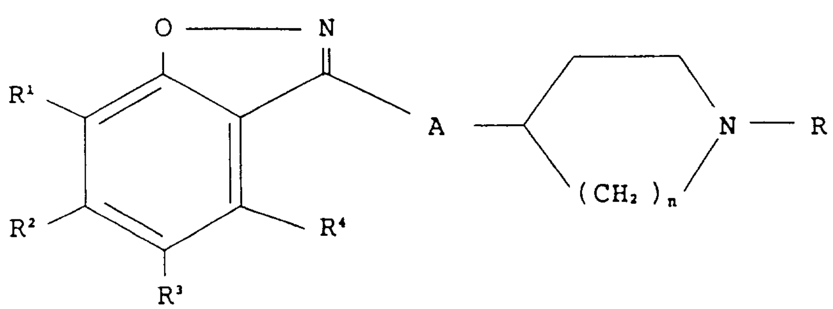

(II)

wherein:

R is hydrogen, alkyl, alkenyl, cycloalkylalkyl, cycloalkylalkenyl, phenylalkyl, phenylalkenyl, naphthylalkyl or naphthylalkenyl;

$R^1$, $R^2$, $R^3$ and $R^4$ are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ or $-COOR^8$, or adjacent groups from $R^1$, $R^2$, $R^3$ and $R^4$ may be bonded to form $-O(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted,

[wherein:

$R^5$ is alkyl, phenyl or phenylalkyl,

$R^6$ and $R^7$ are the same or different and each is hydrogen, alkyl, phenyl, phenylalkyl or a group bonded with the adjacent nitrogen atom to form a heterocyclic ring,

$R^8$ is hydrogen, alkyl, phenyl or phenylalkyl,
$R^9$ is hydrogen, alkyl, phenylalkyl or acyl,
m is 0, 1 or 2, and
p, q, r and s are the same or different and each is 1, 2 or 3];

A is a straight- or branched chain alkylene; and
n is 1, 2 or 3;

wherein:

in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$,$-NHSO_2R^5$, $-CONR^6R^7$, $-NR^6R^7$, $-OCONR^6R^7$, $-OCSNR^6R^7$, $-SO_2NR^6R^7$ and$-COOR^8$ -[wherein $R^5$, $R^6$, $R^7$, $R^8$ and m are as defined above].

**5.** A benzisoxazole compound of Claim 4 or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl.

**6.** A benzisoxazole compound of Claim 4 or a pharmaceutically acceptable salt thereof, wherein R is hydrogen.

**7.** A benzisoxazole compound of Claim 1 or a pharmaceutically acceptable salt thereof, having the formula

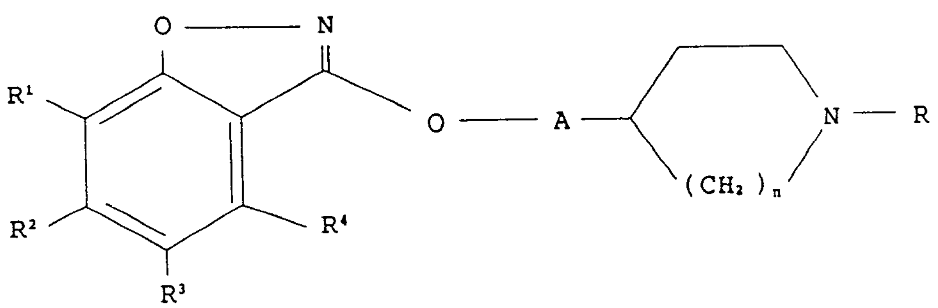

(III)

wherein:

R is hydrogen, alkyl, alkenyl, cycloalkylalkyl, cycloalkylalkenyl, phenylalkyl, phenylalkenyl, naphthylalkyl or naphthylalkenyl;

$R^1$, $R^2$, $R^3$ and $R^4$ are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)-mR^5$, $-NHSO_2R^5$, $-CONR^6R^7$, $-NR^6R^7$, $-OCONR^6R^7$, $-OCSNR^6R^7$, $-SO_2NR^6R^7$ or $-COOR^8$, or adjacent groups from $R^1$, $R^2$, $R^3$ and $R^4$ may be bonded to form $-O(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted,

[wherein:

$R^5$ is alkyl, phenyl or phenylalkyl,
$R^6$ and $R^7$ are the same or different and each is hydrogen, alkyl, phenyl, phenylalkyl or a group bonded with the adjacent nitrogen atom to form a heterocyclic ring,
$R^8$ is hydrogen, alkyl, phenyl or phenylalkyl
$R^9$ is hydrogen, alkyl, phenylalkyl or acyl,
m is 0, 1 or 2, and
p, q, r and s are the same or different and each is 1, 2 or 3];

A is a straight- or branched chain alkylene; and
n is 1, 2 or 3;

wherein:

in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ and $-COOR^8$ - [wherein $R^5$, $R^6$, $R^7$, $R^8$ and m are as defined above].

**8.** A benzisoxazole compound of Claim 7 or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl.

**9.** A benzisoxazole compound of Claim 7 or a pharmaceutically acceptable salt thereof, wherein R is hydrogen.

**10.** A benzisoxazole compound of Claim 1 or a pharmaceutically acceptable salt thereof, having the formula

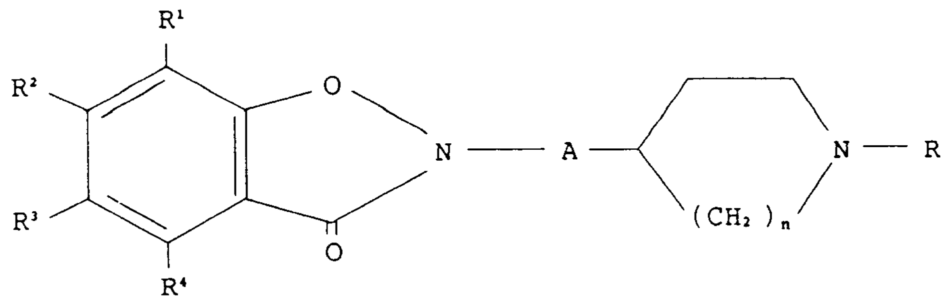

(IV)

wherein:

| | |
|---|---|
| R | is hydrogen, alkyl, alkenyl, cycloalkylalkyl, cycloalkylalkenyl, phenylalkyl, phenylalkenyl, naphthylalkyl or naphthylalkenyl; |
| $R^1$, $R^2$, $R^3$ and $R^4$ | are the same or different and each is hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ or $-COOR^8$, or adjacent groups from $R^1$, $R^2$, $R^3$ and $R^4$ may be bonded to form $-O(CH_2)p-$, $-O(CH_2)qO-$, $-O(CH_2)rN(R^9)-$, $-O(CH_2)sCON(R^9)-$, $-N(R^9)CO-CH=CH-$, benzene ring or heterocyclic aromatic ring, which are optionally substituted,<br>[wherein:<br>$R^5$ is alkyl, phenyl or phenylalkyl,<br>$R^6$ and $R^7$ are the same or different and each is hydrogen, alkyl, phenyl, phenylalkyl or a group bonded with the adjacent nitrogen atom to form a heterocyclic ring,<br>$R^8$ is hydrogen, alkyl, phenyl or phenylalkyl,<br>$R^9$ is hydrogen, alkyl, phenylalkyl or acyl,<br>m is 0, 1 or 2, and<br>p, q, r and s are the same or different and each is 1, 2 or 3]; |
| A | is a straight- or branched chain alkylene; and |
| n | is 1, 2 or 3; |

wherein:

in the above definitions, alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy, heteroaryloxy, benzene ring and heterocyclic aromatic ring may be substituted by 1 to 3 substituents selected from halogen, alkyl, alkoxy, acyl, acyloxy, hydroxyl, nitro, cyano, $-NHCOR^5$, $-S(O)mR^5$, $-NHSO_2R^5$, $-CONR^6R^7$, $-NR^6R^7$, $-OCONR^6R^7$, $-OCSNR^6R^7$, $-SO_2NR^6R^7$ and $-COOR^8$ - [wherein $R^5$, $R^6$, $R^7$, $R^8$ and m are as defined above].

**11.** A benzisoxazole compound of Claim 10 or a pharmaceutically acceptable salt thereof, wherein R is alkyl, alkenyl, cycloalkylalkyl, cycloalkylalkenyl, phenylalkyl, phenylalkenyl, naphthylalkyl or naphthylalkenyl.

**12.** A benzisoxazole compound of Claim 10 or a pharmaceutically acceptable salt thereof, wherein R is hydrogen.

**13.** A benzisoxazole compound of Claim 5 or a pharmaceutically acceptable salt thereof, which is selected from the group consisting of
3-(2-(1-benzyl-4-piperidyl)ethyl)-6,7-dimethoxy-1,2-benzisoxazole,
3-(2-(1-(3-(N-methylcarbamoyloxy)benzyl)-4-piperidyl)ethyl)-6,7-dimethoxy-1,2-benzisoxazole,
6-acetylamino-3-(2-(1-benzyl-4-piperidyl)ethyl)-1,2-benzisoxazole,
3-(2-(1-benzyl-4-piperidyl)ethyl)-6-methoxy-1,2-benzisoxazole,
3-(2-(1-benzyl-4-piperidyl)ethyl)-5-methoxy-1,2-benzisoxazole,
3-(2-(1-benzyl-4-piperidyl)ethyl)-6-(N,N-dimethylthiocarbamoyloxy)-1,2-benzisoxazole,
3-(2-(1-cyclohexylmethyl-4-piperidyl)ethyl)-6,7-dimethoxy-1,2-benzisoxazole,
3-(2-(1-benzyl-4-piperidyl)ethyl)-6-propionylamino-1,2-benzisoxazole,
3-(2-(1-benzyl-4-piperidyl)ethyl)-6-(N-methylacetamino)-1,2-benzisoxazole,
6-(N,N-dimethylamino)-3-(2-(1-benzyl-4-piperidyl)ethyl)-1,2-benzisoxazole,
3-(2-(1-benzyl-4-piperidyl)ethyl)-6-methylamino-1,2-benzisoxazole,
3-(2-(1-(2-chlorobenzyl)-4-piperidyl)ethyl)-6,7-dimethoxy-1,2-benzisoxazole,
3-(2-(1-(4-chlorobenzyl)-4-piperidyl)ethyl)-6,7-dimethoxy-1,2-benzisoxazole,
3-(2-(1-benzyl-4-piperidyl)ethyl)-6-(N,N-dimethylcarbamoyloxy)-1,2-benzisoxazole,
6-acetoxy-3-(2-(1-benzyl-4-piperidyl)ethyl)-1,2-benzisoxazole,
3-(2-(1-benzyl-4-piperidyl)ethyl)-6-(N-methylcarbamoyloxy)-1,2-benzisoxazole,
3-(2-(1-(2-phenylethyl)-4-piperidyl)ethyl)-6,7-dimethoxy-1,2-benzisoxazole,
3-(2-(1-benzyl-4-piperidyl)ethyl)-6-(N-methyl-N-propionylamino)-1,2-benzisoxazole,
6-benzoylamino-3-(2-(1-benzyl-4-piperidyl)ethyl)-1,2-benzisoxazole and pharmaceutically acceptable salts thereof.

**14.** A benzisoxazole compound of Claim 8 or a pharmaceutically acceptable salt thereof, which is selected from the group consisting of
3-(2-(1-cyclohexylmethyl-4-piperidyl)ethyloxy)-5-methyl-1,2-benzisoxazole,
3-(2-(1-(2-phenylethyl)-4-piperidyl)ethyloxy)-1,2-benzisoxazole,
3-(2-(1-cyclohexylmethyl-4-piperidyl)ethyloxy)-6-methyl-1,2-benzisoxazole,
5-methyl-3-(2-(1-(2-phenylethyl)-4-piperidyl)ethyloxy)-1,2-benzisoxazole,
6-chloro-3-(2-(1-(2-phenylethyl)-4-piperidyl)ethyloxy)-1,2-benzisoxazole,
6-methyl-3-(2-(1-(2-phenylethyl)-4-piperidyl)ethyloxy)-1,2-benzisoxazole,
5-methyl-3-(2-(1-benzyl-4-piperidyl)ethyloxy)-1,2-benzisoxazole,
3-(3-(1-benzyl-4-piperidyl)propyloxy)-1,2-benzisoxazole,
3-(2-(1-benzyl-4-piperidyl)ethyloxy)-1,2-benzisoxazole,
3-(2-(1-benzyl-4-piperidyl)ethyloxy)-1,2-benzisoxazole and pharmaceutically acceptable salts thereof.

**15.** A benzisoxazole compound of Claim 11 or a pharmaceutically acceptable salt thereof, which is selected from the group consisting of
2-(2-(1-cyclohexylmethyl-4-piperidyl)ethyl)-5-methyl-1,2-benzisoxazol-3(2H)-one,
5-methyl-2-(2-(1-(2-phenylethyl)-4-piperidyl)ethyl)-1,2-benzisoxazol-3(2H)-one,
2-(2-(1-cyclohexylmethyl-4-piperidyl)ethyl)-6-methyl-1,2-benzisoxazol-3(2H)-one,
6-methyl-2-(2-(1-(2-phenylethyl)-4-piperidyl)ethyl)-1,2-benzisoxazol-3(2H)-one,
6-benzoylamino-2-(2-(1-benzyl-4-piperidyl)ethyl)-1,2-benzisoxazol-3(2H)-one,
6-benzoylamino-2-(3-(1-benzyl-4-piperidyl)propyl)-1,2-benzisoxazol-3(2H)-one and pharmaceutically acceptable salts thereof.

**16.** A pharmaceutical composition comprising, as an active ingredient, the benzisoxazole compound of Claim 2 or a pharmaceutically acceptable salt thereof.

**17.** A pharmaceutical composition comprising, as an active ingredient, the benzisoxazole compound of Claim 5 or a pharmaceutically acceptable salt thereof.

**18.** A pharmaceutical composition comprising, as an active ingredient, the benzisoxazole compound of Claim 8 or a pharmaceutically acceptable salt thereof.

**19.** A pharmaceutical composition comprising, as an active ingredient, the benzisoxazole compound of Claim 11 or a pharmaceutically acceptable salt thereof.

**20.** An acetylcholinesterase inhibitor comprising, as an active ingredient, the benzisoxazole compound of Claim 5 or a pharmaceutically acceptable salt thereof.

**21.** An acetylcholinesterase inhibitor comprising, as an active ingredient, the benzisoxazole compound of Claim 8 or a pharmaceutically acceptable salt thereof.

**22.** An acetylcholinesterase inhibitor comprising, as an active ingredient, the benzisoxazole compound of Claim 11 or a pharmaceutically acceptable salt thereof.

**23.** An agent for the center, comprising, as an active ingredient, the benzisoxazole compound of Claim 5 or a pharmaceutically acceptable salt thereof.

**24.** An agent for the center, comprising, as an active ingredient, the benzisoxazole compound of Claim 8 or a pharmaceutically acceptable salt thereof.

**25.** An agent for the center, comprising, as an active ingredient, the benzisoxazole compound of Claim 11 or a pharmaceutically acceptable salt thereof.

# INTERNATIONAL SEARCH REPORT

International Application No PCT/JP92/01060

**I. CLASSIFICATION OF SUBJECT MATTER** (if several classification symbols apply, indicate all) [6]

According to International Patent Classification (IPC) or to both National Classification and IPC

Int. $Cl^5$ C07D413/06, C07D413/12, C07D413/14, C07D498/04, A61K31/41, A61K31/445, A61K31/535, A61K31/55

**II. FIELDS SEARCHED**

Minimum Documentation Searched [7]

| Classification System | Classification Symbols |
|---|---|
| IPC | C07D413/06, C07D413/12-413/14, A61K31/41-31/43, A61K31/445-31/45, A61K31/535, A61K31/55 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 61-221186 (Janssen Pharm. N.V.), October 1, 1986 (01. 10. 86), & EP, A, 196132 | 1-25 |
| P | EP, A2, 449186 (Du Pont Merck Pharm.), October 2, 1991 (02. 10. 91) | 1-25 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 13, 1992 (13. 11. 92) | December 22, 1992 (22. 12. 92) |
| **International Searching Authority** | **Signature of Authorized Officer** |
| Japanese Patent Office | |